# EUROPEAN PATENT SPECIFICATION

(11) **EP 4 204 539 B1**
(45) Date of publication and mention of the grant of the patent: **27.05.2026**
(21) Application number: 21758717.9
(22) Date of filing: 20.08.2021
(51) Int. Cl.: C12N 1/04, C12N 1/20, C12N 11/04, C12N 11/10

(54) **MICROENCAPSULATION OF MICROBIAL CULTURE USING OCTENYL SUCCINIC ANHYDRIDE STARCH-CHITOSAN COMPLEX COACERVATE**
MIKROVERKAPSELUNG EINER MIKROBIELLEN KULTUR UNTER VERWENDUNG VON OCTENYLBERNSTEINSÄUREANHYDRID-STÄRKE-CHITOSAN-KOMPLEX-KOAZERVAT
MICROENCAPSULATION DE CULTURE MICROBIENNE À L'AIDE D'UN COACERVAT DE COMPLEXE D'AMIDON-CHITOSANE D'ANHYDRIDE OCTÉNYLSUCCINIQUE

(30) Priority: 28.08.2020 IN 202011037173
(43) Date of publication of application: 05.07.2023
(73) Proprietor: Chr. Hansen A/S, 2970 Hoersholm (DK)
(72) Inventor: GADKARI, Pravin Vasant, Mumbai Suburban, Maharashtra 400093 (IN); ALI, Furqan, Mumbai Suburban, Maharashtra 400093 (IN); DHAYAL, Surender Kumar, 2970 Hoersholm (DK)
(74) Representative: NVS EPO Representatives
(86) International application number: PCT/EP2021/073170
(87) International publication number: WO 2022/043223

(56) References cited:
- WO-A1-00/78924
- WO-A1-2010/015580
- WO-A2-2014/006261
- ZHAO M. ET AL: "Probiotic encapsulation in water-in-water emulsion via heteroprotein complex coacervation of type-A gelatin/sodium caseinate", FOOD HYDROCOLLOIDS, vol. 105, 105790, 21 February 2020 (2020-02-21), NL, pages 1 - 8, XP055825228, ISSN: 0268-005X, DOI: 10.1016/j.foodhyd.2020.105790
- OLIVEIRA A. C. ET AL: "Stability of microencapsulated B. lactis (BI 01) and L. acidophilus (LAC 4) by complex coacervation followed by spray drying", JOURNAL OF MICROENCAPSULATION, vol. 24, no. 7, 8 January 2007 (2007-01-08), GB, pages 673 - 681, XP055825209, ISSN: 0265-2048, Retrieved from the Internet <URL:http://dx.doi.org/10.1080/02652040701532908> DOI: 10.1080/02652040701532908
- VANDAMME TH. F. ET AL: "Microencapsulation of probiotics. Introduction to probiotics", ENCAPSULATION AND CONTROLLED RELEASE TECHNOLOGIES IN FOOD SYSTEMS, 2016, pages 97 - 128, XP055788489, Retrieved from the Internet <URL:https://onlinelibrary.wiley.com/doi/pdfdirect/10.1002/9781118946893.ch5> [retrieved on 20210322]
- FANG S. ET AL: "Fabricating multilayer emulsions by using OSA starch and chitosan suitable for spray drying: Application in the encapsulation of [beta]-carotene", FOOD HYDROCOLLOIDS, vol. 93, 1 August 2019 (2019-08-01), NL, pages 102 - 110, XP055856604, ISSN: 0268-005X, DOI: 10.1016/j.foodhyd.2019.02.024

## Description

### FIELD OF THE INVENTION

The present invention relates to microencapsulated microbial cultures with high storage stability and methods for producing these. In particular, the present invention relates to microbial cultures formulated in complex coacervates comprising octenyl succinic anhydride (OSA) starch and chitosan.

### BACKGROUND OF THE INVENTION

In human and animal bodies, microbial cultures, such as lactic acid bacteria (LAB), are the part of normal microbiota. LAB are mostly used as starter cultures in fermented dairy foods and beverages as they can help to improve the nutritional and organoleptic characteristics, as well as extend the shelf life. Some strains of LAB have been reported to exhibit health benefits to human and animals and may thereby be referred to as probiotic strains. The typical process for the production of LAB is through fermentation followed by concentration and freezing of cell biomass. When it comes to applications of LAB, dried powder form produced using freeze drying (FD) is often desired. The dried powders are frequently kept for extended times before utilized in a final application.

Microbial cultures, such as LAB, are very sensitive to different environmental stresses applied during freezing and FD, causing the addition of cryojlyo protectants to be necessary for protecting and retaining viability during processing. Moreover, it is well-known that storage of microbial cultures, such as LAB, at ambient (25-35°C) or higher temperature adversely affect the viability of the microbial cultures. Therefore, storage for an extended period of time necessitates expensive cooling facilitates that are not always available at the point of use.

While there are several concepts on cryo/lyo protectants to shield the LAB during freezing and FD, all of these protectants (ingredients) have limited impact on the storage stability. Therefore, there is an unmet need for methods of protecting microbial cultures during freezing and FD and which at the same time enhance the storage stability of these dried microbial cultures.

Hence, it would be advantageous to provide an improved method for preparing dried microbial cultures that maintain viability after freezing or freeze drying and over extended periods of storage even at elevated temperatures. Specifically, such methods and dry microbial cultures *per se* may be advantageous in the preparation of products which are exposed to conditions of increased environmental stress.

Zhao M. et al. "Probiotic encapsulation in water-in-water emulsion via heteroprotein complex coacervation of type-A gelatin/sodium caseinate", FOOD HYDROCOLLOIDS 105 (2020) 105790 discloses encapsulation of probiotic bacterial cells via heteroprotein complex coacervation of type-A gelatin/sodium caseinate followed by spray drying, to increase shelf stability.

Oliveira A. C. et al. "Stability of microencapsulated B. lactis (BI 01) and L. acidophilus (LAC 4) by complex coacervation followed by spray drying", JOURNAL OF MICROENCAPSULATION, 2007, 24(7):673-681 discloses microencapsulated probiotic bacterial cells by complex coacervation using a casein/pectin complex followed by spray drying, to maintain viability during storage.

### SUMMARY OF THE INVENTION

The present invention relates to a microencapsulation approach for microbial cultures using complex coacervates containing octenyl succinic anhydride (OSA) starch and chitosan. In particular, the present invention discloses methods for producing microencapsulated microbial cultures that endure dry processing and exhibit enhanced storage stability upon storage at even 37°C for extended periods of time. The method relies on complex coacervation in which sequential addition of coacervation components results in the electrostatic formation of a protective complex to shield the entrapped microbial culture. The obtained microencapsulated microbial cultures are well suited for applications in which storage at depressed temperatures is not feasible.

Thus, an object of the present invention relates to the provision of methods for preparing a microbial culture that may be utilized under conditions independent of refrigerated storage.

In particular, it is an object of the present invention to provide an improved method for production of dry microbial cultures that retain cell viability after dry processing and storage at elevated temperatures.

Thus, an aspect of the present invention relates to a microencapsulated microbial culture, said microencapsulated microbial culture comprising;
i) a microbial culture,
ii) a first matrix comprising a first coacervate component, and
iii) a second matrix comprising a second coacervate component,
wherein said first and second coacervate components are octenyl succinic anhydride (OSA) starch or chitosan and wherein said first and second coacervate components are not the same.

In one embodiment, the microbial culture is a bacterium or a yeast. The microbial culture may be, or comprise, a lactic acid bacteria (LAB) of a genus selected from the group consisting of *Lactobacillus, Holzapfelia, Amylolactobacillus, Bifidobacterium, Bombilactobacillus, Companilactobacillus, Lapidilactobacillus, Agrilactobacillus, Schleiferilactobacillus, Loigolactobacilus, Lacticaseibacillus, Latilactobacillus, Dellaglioa, Liquorilactobacillus, Ligilactobacillus, Lactiplantibacillus, Furfurilactobacillus, Paucilactobacillus, Limosilactobacillus, Fructilactobacillus, Acetilactobacillus, Apilactobacillus, Levilactobacillus, Secundilactobacillus, Lentilactobacillus, Leuconostoc, Pediococcus, Lactococcus, Streptococcus, Enterococcus, Bifidobacterium, Brevibacterium,* and *Staphylococcus.* The microbial culture may be a probiotic culture.

In one preferred embodiment, the microencapsulated microbial culture is selected from the group consisting of *Ligilactobacillus animalis* deposited as DSM 33570 at Deutsche Sammlung von Mikroorganismen und Zellkulturen GmbH (DSMZ), *Bifidobacterium animalis* subsp. *lactis* deposited as DSM 15954 at DSMZ, *Streptococcus thermophilus* deposited as DSM 15957 at DSMZ and *Lactococcus lactis* subsp. *lactis* deposited as DSM 21404 at DSMZ.

In one embodiment of the microencapsulated microbial culture, the ratio (wt%/wt%) of OSA starch to chitosan is between 75:25 to 98:2, preferably between 85:15 to 95:5.

In one embodiment of the microencapsulated microbial culture, the ratio (wt%/wt%) of the first and second matrix combined to microbial culture is between 0.5-10.

In one embodiment, the the first or second matrix further comprises an antioxidant, such as trisodium citrate, vitamin C, vitamin E, glutathione, or derivatives thereof.

Another aspect of the present invention relates to a composition comprising the microencapsulated microbial culture as described herein, wherein the composition further comprises one or more additives selected from the group consisting of food-grade ingredients, pharmaceutical ingredients and excipients.

A further aspect of the present invention relates to a product comprising the microencapsulated microbial culture or the composition as described herein, wherein the product is selected from the group consisting of a feed, a plant health product, a food, a beverage and a pharmaceutical product.

An even further aspect of the present invention relates to a method for preparing a microencapsulated microbial culture or a composition as described herein, said method comprising the steps of:
i) mixing a microbial culture with a first matrix comprising a first coacervate component and optionally one or more antioxidants to form a pre-complex solution, and
ii) mixing the pre-complex solution with a second matrix comprising a second coacervate component to form a microencapsulated microbial culture,
wherein said first and second coacervate components are octenyl succinic anhydride (OSA) starch or chitosan and wherein said first and second coacervate components are not the same.

In one embodiment, the microbial culture of step i) of the method according to the above aspect is mixed with the first matrix for a time period in the range of 5 min to 6 hours at a temperature in the range of 4°C to 45°C, and/or wherein mixing of the pre-complex solution with the second matrix in step ii) is carried out for a time period in the range of 5 min to 6 hours at a temperature in the range of 4°C to 45°C.

In one embodiment, the pH in step ii) of the method according to the above aspect is in the range between 6-8, preferably between 6.25-7.5.

In one embodiment, the method according to the above aspect further comprise a step iv) subsequent to step iii), wherein step iv) comprises sublimating water from said frozen microencapsulated microbial culture to obtain a dried microencapsulated microbial culture

Yet another aspect of the present invention relates to a microencapsulated microbial culture or a composition obtainable by a method as described herein.

Still another aspect of the present invention relates to use of a microencapsulated microbial culture or a composition as described herein in a product selected from the group consisting of a feed, a plant health product, a food, a beverage and a pharmaceutical product.

### BRIEF DESCRIPTION OF THE FIGURES

Figure 1 shows complex formation of OSA starch and chitosan at ratios of (A) 85:15 and (B) 95:5. Microscope images are acquired at 20x magnification.
Figure 2 shows *Ligilactobacillus animalis* 51 (LA51) microencapsulated in complex coacervates of OSA starch:chitosan at ratios of (A) 85:15 and (B) 95:5. Microscope images are acquired at 100x magnification.
Figure 3 shows the viability of freeze-dried granulates of *Ligilactobacillus animalis* 51 (LA51) encapsulated in either OSA starch-chitosan complex coacervates (ratios 85:15 or 95:5), the reference cryoprotectant or a control containing chitosan and trisodium citrate (5 wt%/wt%).
Figure 4 shows the reduction in viability of freeze-dried granulates of *Ligilactobacillus animalis* 51 (LA51) encapsulated in either OSA starch-chitosan complex coacervates (ratios 85:15 or 95:5) or the reference cryoprotectant, upon storage in 4 weeks at A_{w} ≤ 0.15, T=37°C.
Figures 5 to 8 are graphs showing reduction in viability of freeze-dried granulates of bacteria according to various embodiments, encapsulated in either octenyl succinic anhydride (OSA) starch-chitosan complex coacervates (ratios 85:15 or 95:5) or the reference cryoprotectant or No cryoprotectant, upon storage in 12 weeks at a_{w} ≤ 0.05, T=37°C.

The present invention will in the following be described in more detail.

### DETAILED DESCRIPTION OF THE INVENTION

### Definitions

Prior to outlining the present invention in more details, a set of terms and conventions is first defined:

### Microbial culture

In the present context, the term "microbial culture" refers to a population of microorganisms. Microorganisms include all unicellular organisms, such as archaea and bacteria, but also many multicellular organisms, such as fungi and algae.

### Probiotic culture

In the present context, the terms "probiotic" or "probiotic culture" refers to microbial cultures which, when ingested in the form of viable cells by humans or animals, confer an improved health condition, e.g. by suppressing harmful microorganisms in the gastrointestinal tract, by enhancing the immune system or by contributing to the digestion of nutrients. Probiotics may also be administered to plants. Probiotic cultures may comprise bacteria and/or fungi.

### Lactic acid bacteria (LAB)

In the present context, the term "lactic acid bacteria (LAB)" refers to a group of Gram positive, catalase negative, non-motile, microaerophilic or anaerobic bacteria that ferment sugar with the production of acids including lactic acid as the predominantly produced acid, acetic acid, formic acid and propionic acid. The industrially most useful lactic acid bacteria include, but are not limited to, *Lactococcus species (spp.), Streptococcus spp., Lactobacillus spp., Leuconostoc spp., Pediococcus spp., Brevibacterium spp, Enterococcus spp. and Propionibacterium spp.* Additionally, lactic acid producing bacteria belonging to the group of the strict anaerobic bacteria, *Bifidobacteria,* i.e. *Bifidobacterium* spp. which are frequently used as food starter cultures alone or in combination with lactic acid bacteria, are generally included in the group of lactic acid bacteria. Even certain bacteria of the genus *Staphylococcus (e.g. S. carnosus, S. equorum, S. sciuri, S. vitulinus and S. xylosus*) have been referred to as LAB (Seifert & Mogensen (2002)).

### Viability

In the present context, the term "viability" refers to living cells in a culture. Thus, the viability of a cell culture may be determined by measuring the number of colony forming units (CFU). CFU refer to the number of individual colonies of any microbe that grow on a plate of media. This value in turn represents the number of bacteria or fungi capable of replicating as they have formed colonies on the plate.

In brief, the CFU/g can be determined as follows; A known amount of sample (e.g. freeze dried) is homogenized with a specific volume of diluent (1:100), using a stomacher, the solution is then resuspended by using a vortex mixer and is then subjected to decimal dilutions in peptone saline diluent (also referred to as 'maximum recovery diluent (MRD)'). MRD comprises peptone, NaCl and demineralised water. Dilutions are poured on the plates, mixed with MRS Agar (Hi-media, M641) and incubated. After incubation, colonies are counted manually.

Particularly, stability of a sample is assessed by counting the colony-forming units (CFU) per gram, using the following assay. Viable cell counts are determined in freeze-dried granulates sampled immediately after freeze-drying and at selected time points during the stability studies. A standard pour-plating method is used. The freeze-dried material is suspended in sterile peptone saline diluent (BD DifcoTM Lactobacilli MRS Agar, Fisher Scientific) and homogenized by stomaching using stomacher (bioMérieux, Inc. Durham, NC). After 30 minutes of revitalization, stomaching is repeated and the cell suspension is serially diluted in peptone saline diluent. For the cfu of *Bifidobacterium animalis* subsp. *lactis* deposited as DSM 15954 (BB-12^{®}), the dilutions are plated in duplicates on MRS agar (BD DifcoTM Lactobacilli MRS Agar, Fisher Scientific) supplemented with 0.5g/L of L-cysteine hydrochloride (Sigma-Aldrich, Inc.). The agar plates are incubated anaerobically for three days at 37°C. For the cfu of *Ligilactobacillus animalis* LA51, deposited as DSM 33570, the dilutions are plated in duplicates on MRS agar (BD DifcoTM Lactobacilli MRS Agar, Fisher Scientific). The agar plates are incubated anaerobically for three days at 37°C. In case of *Streptococcus thermophilus* TH4 (DSM 15957) cfu, the dilutions are plated in duplicates on M17 agar (BD DifcoTM Lactobacilli MRS Agar, Fisher Scientific) supplemented with 0.5g/L of monosodium phosphate (Sigma-Aldrich, Inc.) and 0.5g/L of disodium phosphate (Sigma-Aldrich, Inc.). The agar plates are incubated aerobically for three days at 37°C. For the cfu of *Lactococcus lactis* subsp. *lactis* R607 (DSM 21404), the dilutions are plated in duplicates on M17 agar (BD DifcoTM Lactobacilli MRS Agar, Fisher Scientific) supplemented with 0.5g/L of monosodium phosphate (Sigma-Aldrich, Inc.) and 0.5g/L of disodium phosphate (Sigma-Aldrich, Inc.). The agar plates are incubated aerobically for three days at 37°C. Plates with 30 - 300 colonies are chosen for counting of colony forming units (CFU). The result is reported as average CFU/g freeze-dried sample, calculated from the duplicates.

### Microencapsulated

In the present context, the term "microencapsulated" refers to an entity, which on a micrometric scale are secluded from the surrounding environment. Thus, a microencapsulated microbial culture is a microbial culture which are compartmentalized into distinct entities separated from each other and the medium into which they are dispersed.

### Complex coacervate

In the present context, the term "complex coacervate" refers to an aqueous phase (or droplet) rich in the microbial culture that is formed upon complex coacervation using two or more biopolymers of opposite charge. The complex coacervate forms due to liquid-liquid phase separation and is a dense phase that exist in equilibrium with a dilute phase. Thus, the complex coacervate may be characterized as a lyophilic colloid.

The method of complex coacervation involves the mixing of an entity to be encapsulated, such as a microbial culture, with at least two biopolymers of opposite charge. Herein, the biopolymers of opposite charge are referred to as coacervate components and comprised in a first and second matrix, respectively.

### Chitosan

In the present context, the term "chitosan" refers to a linear polysaccharide composed of randomly distributed *β* -(1→4)-linked D-glucosamine (deacetylated unit) and N-acetyl-D-glucosamine (acetylated unit). Chitosan is produced by deacetylation of chitin and the degree of acetylation ranges from 60% to 100%. Thus, the term chitosan as used herein includes variants with different degree of deacetylation. Moreover, the term chitosan encompasses chitosan with a molecular weight in the range of 3 kDa to 20 kDa.

### Octenyl succinate anhydride (OSA) starch

In the present context, the term "octenyl succinate anhydride (OSA) starch" refers to starch substituted with n-octenyl succinic anhydride and all derivatives thereof. OSA starch is anionic due to a carboxyl group and hydrophobic due to the C8-alkene chain. OSA starch may be produced as derivatives having varying degree of substitution. Thus, the term OSA starch as used herein includes derivatives with different degree of substitution, such as a degree of substation up to 0.11, more preferably up to 0.03. The OSA starch may also be referred to as n-octenyl succinate anhydride (nOSA) starch.

The terms "octenyl succinate anhydride (OSA) starch" and "starch octenyl succinate" are used interchangeably herein. Both corresponds to INS 1450, E1450.

The source of starch to be substituted may be varied, and include, but are not limited to, maize, rice, potato, tapioca and pearl millet.

### Antioxidant

In the present context, the term "antioxidant" refers to a compound that inhibit oxidation. The antioxidant may be industrial chemicals or natural compounds. As used herein, antioxidants include, but are not limited to, trisodium citrate, vitamin C, vitamin E, glutathione and derivatives thereof.

It is to be understood that antioxidants as used herein include mineral salts of vitamin C, such as sodium ascorbate. Also, the vitamin E is to be understood as including all variants of tocopherols and tocotrienols (alpha, beta, gamma, delta).

### Hydrophobic coating

In the present context, the term "hydrophobic coating" refers to a hydrophobic layer or shell that is positioned on the surface of the complex coacervate. Such hydrophobic layer or shell may comprise one or more hydrophobic compounds or molecules comprising a hydrophobic moiety that cause the outer surface of the complex coacervate to be hydrophobic.

The hydrophobic coating may comprise, but is not limited to, fats and waxes, such as carnauba wax, beeswax, coco butter fat, hydrogenated palm oil, palm stearin, shea butter fat, mango butter fat, soya oil, olive oil, coconut oil, rice bran oil, sunflower oil, candelilla wax, rice bran wax and laurel wax.

### Food-grade ingredient

In the present context, the term "food-grade ingredient" refers to any compound that is non-toxic and safe for consumption and comply with the Food Chemicals Codex (FCC). Food-grade ingredients include, but are not limited to, compounds that can alter attributes such as aroma, flavour, acidity, colour, viscosity and texture, as well as preservatives, nutrients, thickeners, sweeteners and emulsifiers.

Preferred food-grade ingredients include, but are not limited to, lactose, maltodextrin, whey protein, casein, corn starch, dietary fibres, gums and gelatine.

### Pharmaceutical ingredient

In the present context, the term "pharmaceutical ingredient" refers to an ingredient in a pharmaceutical formulation that is not an active ingredient.

Pharmaceutical ingredients include, but are not limited to, calcium carbonate, sodium carboxymethyl cellulose, talc, polydimethylsiloxane, hydroxypropyl cellulose and hydroxypropyl methylcellulose.

### Excipient

In the present context, the term "excipient" refers to a natural or synthetic substance formulated alongside the active ingredient or pharmaceutical ingredient (an ingredient that is not the active ingredient) of a medication, included for the purpose of stabilization, bulking, or to confer a therapeutic enhancement on the active ingredient in the final dosage form, such as facilitating drug absorption, reducing viscosity, enhancing solubility, adjusting tonicity, mitigating injection site discomfort, depressing the freezing point, or enhancing stability.

Excipients include, but are not limited to, microcrystalline cellulose, titanium dioxide and aluminium silicate.

### Storage stability

In the present context, the term "storage stability" refers to the ability of a microencapsulated microbial culture to maintain viability when stored at accelerated storage conditions over an extended duration of time, such as at a temperature of 37°C and a water activity (A_{w}) ≤ 0.15 for a period of 4 weeks. A_{w} of a food is the ratio between the vapor pressure of the microencapsulated microbial culture itself, when in a completely undisturbed balance with the surrounding air media, and the vapor pressure of distilled water under identical conditions. In the present context A_{w} is measured either by a resistive electrolytic, a capacitance or a dew point hygrometer.

Storage stability can be determined by analysing how the count of viable microbial cells develop over time. Viability of the microbial culture is measured by determining the CFU/g as described herein. Thus, a measure of the storage stability of the microencapsulated microbial culture may be determined by evaluating CFU/g of the dry granulates of microencapsulated microbial culture at time point 0 (just after drying) and after 4 weeks of storage at accelerated storage conditions.

In brief, the storage stability of FD granulates or FD grinded powder is investigated as follows; A sample of FD granulates of microbial culture (60 mesh grinded powder) is blended in CaCO₃ to achieve a sample with a water activity (A_{w}) of 0.15. The sample is placed in an aluminium bag and the bag is sealed so that no air is trapped within it. The bag is stored at 37°C for 4 weeks and the CFU/g is determined for the sample.

### Microencapsulated microbial cultures, compositions comprising the same and methods for their production

Microbial cultures, such as lactic acid bacteria (LAB), play key parts in many fermented products, in which they add nutritional value to the product and improve the organoleptic and textural profile of e.g. food products. The microbial cultures are typically acquired separately as powdered compositions and mixed with additional ingredients to yield a final product. Thus, the powdered composition comprising the microbial culture need as a minimum to maintain viability from the point of becoming a dried granulate to the point at which the powdered microbial cultures is included in a final product. Ideally, the microbial cultures are kept refrigerated during transport, supplementary processing and as part of the final product. However, this is not always possible as cold transport and storage is both expensive and, in many cases, not feasible in e.g. developing countries or remote regions. Moreover, the final product may be an article that is not readily stored under refrigerated conditions. This is typically the case of animal feed.

To deliver microbial cultures of high quality, e.g. high viability, under such environmental stress conditions, it is a necessary to decrease yield loss during downstream processing and eliminate the requirement of refrigerated transport and storage. However, no methods exist that both provide adequate cryo/lyo protection and enhance storage stability at elevated temperatures.

Herein are provided methods for microencapsulation of microbial cultures in complex coacervates that form based on liquid-liquid phase separation and electrostatic interactions between the coacervate components. The identification of octenyl succinic anhydride (OSA) starch and chitosan as suitable components of first and second matrices for encapsulation of microbial cultures led to the provision of advantageous microencapsulated microbial cultures and compositions comprising the same that maintain viability even over extended periods of storage at elevated temperatures.

Thus, an aspect of the present invention relates to a microencapsulated microbial culture, said microencapsulated microbial culture comprising:
i) a microbial culture,
ii) a first matrix comprising a first coacervate component, and
iii) a second matrix comprising a second coacervate component,
wherein said first and second coacervate components are octenyl succinic anhydride (OSA) starch or chitosan and wherein said first and second coacervate components are not the same.

The first or second matrix may further comprise one or more antioxidant(s).

The coacervation components interact electrostatically in aqueous medium to entrap the microbial culture in the form of dense phase droplets. The inclusion of OSA starch and chitosan in the first and second matrices is interchangeable, meaning that either OSA starch or chitosan may be included in the first matrix. However, both coacervate components must be present for the complex coacervation process to unfold.

An embodiment of the present invention relates to the microencapsulated microbial culture as described herein, wherein said first coacervate component is octenyl succinic anhydride (OSA) starch and said second coacervate component is chitosan.

It is the selection of coacervate component that drives the microencapsulation process. Thus, the microencapsulation technique by complex coacervation as presented herein is not limited to a specific type of microbial culture but is a general microencapsulation concept. Thus, it is contemplated that any type of microbial culture may advantageously be microencapsulated as described herein.

Two types of microorganisms that are of great importance in many consumer goods are bacteria and yeast. These microorganisms are included e.g. in fermented food, feed mixes and nutritional supplements, wherein their health benefits are well-documented.

Therefore, an embodiment of the present invention relates to the microencapsulated microbial culture as described herein, wherein the microbial culture is a bacterium or a yeast.

Another embodiment of the present invention relates to the microencapsulated microbial culture as described herein, wherein the microbial culture is or comprises a genus selected from the group consisting of *Lactobacillus, Leuconostoc, Pediococcus, Lactococcus, Streptococcus, Enterococcus, Bifidobacterium, Propionibacterium, Brevibacterium, Staphylococcus, Bacillus* and *Saccharomyces.*

Of particular interest are lactic acid bacteria (LAB) that are an order of Gram-positive bacteria sharing common metabolic and physiological characteristics. LAB produce lactic acid as the major metabolic outcome of carbohydrate fermentation. Ever since it was discovered that acidification by food fermentation could preserve food by inhibiting growth of spoilage agents, LAB has been utilized purposefully in food fermentation. However, since efficient food fermentation requires high quality viable microorganisms, the development of fermented foods has been halted in areas that do not have advanced facilities to handle the fragile microorganisms.

Specifically, microbial cultures, such as LAB, are not easily handled in some developing countries or remote regions due to the requirement and cost of refrigerated facilities. The microencapsulated microbial cultures described herein tolerate storage at elevated temperatures and may thus open up development of products containing microbial cultures, such as LAB, to a broader ensemble of product developers.

Thus, an embodiment of the present invention relates to the microencapsulated microbial culture as described herein, wherein the microbial culture is a lactic acid bacteria (LAB). Another embodiment of the present invention relates to the microencapsulated microbial culture as described herein, wherein the microbial culture is or comprises a lactic acid bacteria (LAB) of a genus selected from the group consisting of *Lactobacillus, Holzapfelia, Amylolactobacillus, Bombilactobacillus, Companilactobacillus, Lapidilactobacillus, Agrilactobacillus, Schleiferilactobacillus, Loigolactobacilus, Lacticaseibacillus, Latilactobacillus, Dellaglioa, Liquorilactobacillus, Ligilactobacillus, Lactiplantibacillus, Furfurilactobacillus, Paucilactobacillus, Limosilactobacillus, Fructilactobacillus, Acetilactobacillus, Apilactobacillus, Levilactobacillus, Secundilactobacillus and Lentilactobacillus, Leuconostoc, Pediococcus, Lactococcus, Streptococcus, Enterococcus, Bifidobacterium, Brevibacterium,* and *Staphylococcus.*

It will be appreciated that the *Lactobacillus* genus taxonomy was updated in 2020. The new taxonomy is disclosed in Zheng et al. 2020 and will be cohered to herein if nothing else is noticed. For the purpose of the present invention, table 1 presents a list of new and old names of some *Lactobacillus* species relevant to the present invention.

**Table 1. New and old names of some Lactobacillus species relevant to the present invention**

| **Old Name** | **New Name** |
|---|---|
| *Lactobacillus reuteri* | *Limosilactobacillus reuteri* |
| *Lactobacillus rhamnosus* | *Lacticaseibacillus rhamnosus* |
| *Lactobacillus salivarius* | *Ligilactobacillus salivarius* |
| *Lactobacillus casei* | *Lacticaseibacillus casei* |
| *Lactobacillus paracasei* subsp. *paracasei* | *Lacticaseibacillus paracasei* subsp. *Paracasei* |
| *Lactobacillus plantarum* subsp. *plantarum* | *Lactiplantibacillus plantarum* subsp. *plantarum* |
| *Lactobacillus fermentum* | *Limosilactobacillus fermentum* |
| *Lactobacillus animalis* | *Ligilactobacillus animalis* |
| *Lactobacillus buchneri* | *Lentilactobacillus buchneri* |
| Lactobacillus curvatus | *Latilactobacillus* curvatus |
| *Lactobacillus futsaii* | *Companilactobacillus futsaii* |
| *Lactobacillus sakei* subsp. *sakei* | *Latilactobacillus sakei* subsp. |
| *Lactobacillus pentosus* | *Lactiplantibacillus pentosus* |

Bacteria of the *Lactobacillus* genus, as well as the related newly updated genera, have for a long time been known to constitute a significant component of the microbiota in the human body, such as in the digestive system, urinary system and genital system. For this reason, these bacteria have been heavily utilized in in health and/or nutritional products aimed at aiding, maintaining or restoring the natural balance of microbiota in the human body. Examples of application of *Lactobacillus* include treatment or amelioration of diarrhea, vaginal infections, and skin disorders such as eczema.

Thus, an embodiment of the present invention relates to the microencapsulated microbial culture as described herein, wherein the microbial culture is or comprises a lactic acid bacteria (LAB) of a genus selected from the group consisting of *Lactobacillus, Limosilactobacillus, Lacticaseibacillus, Ligilactobacillus, Lacticaseibacillus, Lacticaseibacillus, Lactiplantibacillus, Limosilactobacillus, Ligilactobacillus, Lentilactobacillus, Latilactobacillus, Companilactobacillus, Latilactobacillus* and *Lactiplantibacillus.* Another embodiment of the present invention relates to the microencapsulated microbial culture as described herein, wherein the microbial culture is of a species of *Limosilactobacillus reuteri, Lacticaseibacillus rhamnosus, Ligilactobacillus salivarius, Lacticaseibacillus casei, Lacticaseibacillus paracasei subsp. paracasei, Lactiplantibacillus plantarum subsp. plantarum, Limosilactobacillus fermentum, Ligilactobacillus animalis, Lentilactobacillus buchneri, Latilactobacillus curvatus, Companilactobacillus futsaii, Latilactobacillus sakei subsp., Lactiplantibacillus pentosus, Lactobacillus acidophillus, Lactobacillus helveticus, Lactobacillus gasseri* and *Lactobacillus delbrueckii.*

Yet another embodiment of the present invention relates to the microencapsulated microbial culture as described herein, wherein the microbial culture is or comprises at least one selected from the group consisting of:
- *Bifidobacterium animalis* subsp. *lactis* deposited as DSM 15954 at Deutsche Sammlung von Mikroorganismen und Zellkulturen GmbH (DSMZ) by Chr. Hansen A/S, Hørsholm, Denmark on 30 September 2003,
- *Bifidobacterium animalis* subsp. *lactis* deposited as ATCC 27536 and publicly available through the ATCC collection (https://www.lgcstandards-atcc.org/en.aspx),
- *Biliclobacterium animalis* subsp. *lactis* deposited as DSM 10140 and publicly available through the DSMZ collection (https://www.dsmz.de/),
- *Lactobacillus acidophilus* deposited as DSM 13241 at Deutsche Sammlung von Mikroorganismen und Zellkulturen GmbH (DSMZ) by Chr. Hansen A/S, Hørsholm, Denmark on 20 January 2000,
- *Lactobacillus rhamnosus* deposited as ATCC 53103 and publicly available through the ATCC collection (https://www.lgcstandards-atcc.org/en.aspx),
- *Lactobacillus rhamnosus* deposited as ATCC 55826 and publicly available through US patent 6,479,051,
- *Lactobacillus reuteri* deposited as ATCC 55845 and publicly available through US patent 6,479,051,
- *Lactobacillus paracasei* subsp. *paracasei* deposited as ATCC 55544 and publicly available through US patent 6,033,091,
- *Lactobacillus paracasei* deposited as LMG-17806 and publicly available through US patent 6,599,504,
- *Streptococcus thermophilus* deposited as DSM 15957 at Deutsche Sammlung von Mikroorganismen und Zellkulturen GmbH (DSMZ) by Chr. Hansen A/S, Hørsholm, Denmark on 30 September 2003,
- *Lactobacillus fermentum* deposited as NM02/31074 and publicly available through European patent EP 1539927,
- *Lactobacillus paracasei* subsp. *paracasei* deposited as CCTCC M204012 and publicly available through US patent 6,599,504, and
- *Ligilactobacillus animalis* deposited as DSM 33570 at Leibniz Institute DSMZ-German Collection of Microorganisms and Cell cultures Inhoffenstr. 7B, 38124 Braunschweig Germany (Deutsche Sammlung von Mikroorganismen und Zellkulturen (DSMZ) 5 GmbH1 Inhoffenstr. 7B, D-38124 Braunschweig, Germany) by Chr. Hansen A/S, Hørsholm, Denmark on 8 July 2020,
- *Lactococcus lactis* subsp. *lactis* deposited as DSM 21404 deposited_at Deutsche Sammlung von Mikroorganismen und Zellkulturen GmbH (DSMZ) by Chr. Hansen A/S, Hørsholm, Denmark on 23 April 2008.

The applicant requests that a sample of the deposited microorganisms stated above may only be made available to an expert, subject to available provisions governed by Industrial Property Offices of States Party to the Budapest Treaty, until the date on which the patent is granted.

For US patents, the Budapest Treaty provides that any restriction of public access to samples of deposited biological material must be irrevocably removed as of the date of grant of the relevant patent. Accordingly, the above strains are publicly available because they have been deposited under the Budapest Treaty and a US patent referring to the strain has issued.

For EP patents, the Budapest Treaty provides that if no expert solution has been requested the deposited biological material becomes available upon request to any person from the date of publication of the European patent application.

If an expert solution has been requested, restrictions concerning the furnishing of samples apply. Information as to a request for an expert solution is published on the front page of an issued patent.

There is no indication on the front pages of EP 1359924 or EP 1539927 (referred to above) that a request for expert solution has been made. Thus, *Bifidobacterium animalis* subsp *lactis* deposited as ATCC 27536 referred to in EP 1359924 and *Lactobacillus fermentum* deposited as NM02/31074 referred to in EP 1539927 are publicly available.

A further embodiment of the present invention relates to the microencapsulated microbial culture as described herein, wherein the microbial culture is or comprises *Ligilactobacillus animalis* 51 (LA51) deposited as DSM 33570 at Leibniz Institute DSMZ-German Collection of Microorganisms and Cell cultures Inhoffenstr. 7B 38124 Braunschweig Germany (Deutsche Sammlung von Mikroorganismen und Zellkulturen (DSMZ) 5 GmbH1 Inhoffenstr. 7B, D-38124 Braunschweig, Germany) by Chr. Hansen A/S, Hørsholm, Denmark on 8 July 2020.

A further embodiment of the present invention relates to the microencapsulated microbial culture as described herein, wherein the microbial culture is or comprises *Bifidobacterium animalis* subsp. *lactis* deposited as DSM 15954 at DSMZ.

A further embodiment of the present invention relates to the microencapsulated microbial culture as described herein, wherein the microbial culture is or comprises *Streptococcus thermophilus* deposited as DSM 15957 at DSMZ.

A further embodiment of the present invention relates to the microencapsulated microbial culture as described herein, wherein the microbial culture is or comprises *Lactococcus lactis* subsp. *lactis* deposited as DSM 21404 at DSMZ.

Probiotic culture are cultures of live microorganisms, which upon ingestion by a subject provide health benefits to the subject. Products comprising probiotic cultures include dairy products, animal feed and beverages. Thus, it is to be understood that the microencapsulated microbial cultures described herein may be administered not only to humans but also animals, and even plants.

Thus, an embodiment of the present invention relates to the microencapsulated microbial culture as described herein, wherein the microbial culture is a probiotic culture.

The process of complex coacervation is driven by the coacervate components OSA starch and chitosan. The two coacervate components may be included in varying amounts and ratios. It has been found that complex coacervates with a predominant fraction of OSA starch compared to chitosan result in better cryo/lyo protection and enhanced storage stability. Thus, variants of complex coacervates with high content of OSA starch are preferred.

Therefore, an embodiment of the present invention relates to the microencapsulated microbial culture as described herein, wherein the content of OSA starch in the composition is between 20-40 wt%, such as between 25-37 wt%, preferably between 30-35 wt%.

Another embodiment of the present invention relates to the microencapsulated microbial culture as described herein, wherein the content of chitosan in the composition is between 1-10 wt%, such as between 2-8 wt%, preferably between 4- 6 wt%.

A further embodiment of the present invention relates to the microencapsulated microbial culture as described herein, wherein the ratio (wt%/wt%) of OSA starch to chitosan is between 75:25 to 98:2, preferably between 85:15 to 95:5. A still further embodiment of the present invention relates to the microencapsulated microbial culture as described herein, wherein the ratio (wt%/wt%) of OSA starch to chitosan 15 is 85:15 or 95:5.

Oxidation is the loss of electrons of an atom or ion. In the present context, oxidation refers to oxidation of molecular oxygen and means that oxygen is metabolised to unstable free radicals, which can pry away electrons from other molecules. Oxidation may therefore lead to damaging of cell membranes and other cellular components, such as proteins, lipids and DNA. To avoid damage to the microencapsulated microbial culture, one or more antioxidants are included in the first matrix to prevent oxidation. The antioxidants may be of either natural or synthetic origin.

Therefore, an embodiment of the present invention relates to the microencapsulated microbial culture as described herein, wherein the one or more antioxidants are selected from the group consisting of trisodium citrate, sodium ascorbate, vitamin E and combinations thereof.

Another embodiment of the present invention relates to the microencapsulated microbial culture as described herein, wherein the one or more antioxidants is trisodium citrate.

The content of antioxidants may be tailored to the specific microbial culture to be encapsulated and the expected application of the final powdered product. If the microencapsulated microbial culture is intended for an application for which extended storage is expected, it may for instance be preferable to increase the content of antioxidants. The content of antioxidants may also be adjusted depending on the conditions of storage, e.g. the degree of exposure to air.

Therefore, an embodiment of the present invention relates to the microencapsulated microbial culture as described herein, wherein the content of antioxidants in the composition is between 2-20 wt%, such as between 5-15 wt%, preferably between 8-12 wt%.

The ratio (wt%/wt%) of matrix material to microbial culture in the formulation may be customized to suit the specific application. This ratio is also referred to as the encapsulation index (EI). Without being bound by theory, it is contemplated that microencapsulated microbial cultures formulated at high EI will more efficiently restrict absorption of water and thereby yield increased storage stability. However, increasing the content of matrix material will dilute the content of microbial culture in the final product. Consequently, the optimal balance between matrix material and microbial culture is decided also by factors such as the time scale of storage and storage conditions, which may vary depending on the intended application.

Therefore, an embodiment of the present invention relates to the microencapsulated microbial culture as described herein, wherein the ratio (wt%/wt%) of the first and second matrix combined to microbial culture is between 0.5-10.

Another embodiment of the present invention relates to the microencapsulated microbial culture as described herein, wherein the ratio (wt%/wt%) of the first and second matrix combined to microbial culture is 1.

The complex coacervation process is a result of the interactions of oppositely charged coacervate components in aqueous medium. The charge of the coacervate components depends on the pKa of any available reactive groups. The pKa of the carboxylic acid moiety on 3-octenoic acid of OSA starch is ~ 5, which means that the carboxylic acid moiety exists mainly in its deprotonated form at e.g. pH 6 and above. In contrast, the amino groups of chitosan have a pKa value of ~6.5 and protonation is therefore significant at pH 6. By varying the pH around the pKa of the amino groups of chitosan it is possible to alter the interaction between the coacervation components. Moreover, the interaction depends on the ratio (wt%/wt%) of OSA starch to chitosan.

Thus, an embodiment of the present invention relates to the microencapsulated microbial culture as described herein, wherein the pH is in the range between 6-8, preferably between 6.25-7.5.

Another embodiment of the present invention relates to the microencapsulated microbial culture as described herein, wherein the pH is in the range of 6 to 7, such as in the range of 6.25 to 6.75, and the ratio (wt%/wt%) of OSA starch to chitosan is in the range of 90:10 to 99: 1, such as in the range of 92:8 to 98:2.

Yet another embodiment of the present invention relates to the microencapsulated microbial culture as described herein, wherein the pH is in the range of 6.75 to 8, such as in the range of 7 to 7.5, and the ratio (wt%/wt%) of OSA starch to chitosan is in the range of 70:30 to 90: 10, such as in the range of 80:20 to 87:13.

It is appreciated that the interaction between chitosan and OSA starch will also be affected by the extent of deacetylation of the chitosan and the extent of n-octenyl succinic anhydride substitutions on the starch.

Storage stability may be further increased by applying a hydrophobic coating on the exterior of the microencapsulated microbial culture. Thus, an embodiment of the present invention relates to the microencapsulated microbial culture as described herein further comprising a hydrophobic coating. Another embodiment of the present invention relates to the microencapsulated microbial culture as described herein, wherein the hydrophobic coating comprises one or more fats or waxes and mixture thereof. A further embodiment of the present invention relates to the microencapsulated microbial culture as described herein, wherein the hydrophobic coating comprises one or more ingredients selected from group consisting of carnauba wax, beeswax, coco butter fat, hydrogenated palm oil, palm stearin, shea butter fat, mango butter fat, soya oil, olive oil, coconut oil, rice bran oil, sunflower oil, candelilla wax, rice bran wax and laurel wax.

The combination of coacervate components and careful selection of process parameters has resulted in microencapsulated microbial cultures that readily withstand drying and storage at harsh conditions for extended periods of time. The count of viable microbes after storage is the most important product quality to the downstream developer or consumer. Thus, an embodiment of the present invention relates to the microencapsulated microbial culture as described herein, wherein the microbial culture comprises a content of viable microbes in the range from 10⁶ - 10¹² CFU/g after storage for 4 weeks at 37°C and A_{w} ≤ 0.15. Another embodiment of the present invention relates to the microencapsulated microbial culture as described herein, wherein the microbial culture comprises a content of viable microbes of at least 10⁶ CFU/g, such as at least 10⁷ CFU/g, such as at least 10⁸ CFU/g, preferably at least 10⁹ CFU/g, more preferably at least 10¹⁰ CFU/g after storage for 4 weeks at 37°C and A_{w} ≤ 0.15.

The microencapsulated microbial culture will typically be utilized as an additive in an end product comprising also other ingredients. It is therefore contemplated that the microencapsulated microbial culture for many applications will be part of a more complex composition. Thus, an aspect of the present invention relates to a composition comprising the microencapsulated microbial culture as described herein.

As the microencapsulated microbial culture is admixed with other ingredients the complex coacervates formed during the mixing of OSA starch and chitosan stay intact to safeguard the microbial culture from any environment potentially harmful to the microorganisms. Therefore, an embodiment of the present invention relates to the composition as described herein, wherein the composition comprises complex coacervates comprising the microbial culture, the first matrix and the second matrix.

The order of addition of coacervate components may affect the arrangement of the components within complex coacervate. Therefore, an embodiment of the present invention relates to the composition as described herein, wherein the complex coacervates are arranged with an interior part comprising the microbial culture and the first matrix, and an exterior part comprising the second matrix.

The microencapsulated microbial culture may be utilized for many different types of applications spanning from e.g. health products, nutritional supplement and pharmaceutics to animal feed. Thus, a composition encompassing the microencapsulated microbial culture may a wide range of additives. Therefore, an embodiment of the present invention relates to the composition as described herein, wherein the composition further comprises one or more additives selected from the group consisting of food-grade ingredients, pharmaceutical ingredients and excipients.

Another embodiment of the present invention relates to the composition as described herein, wherein the food-grade ingredients are selected from the group consisting of lactose, maltodextrin, whey protein, casein, corn starch, dietary fibres, gums and gelatine.

A further embodiment of the present invention relates to the composition as described herein, wherein the pharmaceutical ingredients are selected from the group consisting of calcium carbonate, sodium carboxymethyl cellulose, talc, polydimethylsiloxane, hydroxypropyl cellulose and hydroxypropyl methylcellulose.

Yet another embodiment of the present invention relates to the composition as described herein, wherein the excipients are selected from the group consisting of microcrystalline cellulose, titanium dioxide and aluminium silicate.

Many additives are provided in dry form to extent the shelf life and ease the handling of the additive. The microencapsulated microbial culture is no exception and is also provided in dry form. Therefore, an embodiment of the present invention relates to the microencapsulated microbial culture as described herein, wherein the microencapsulated microbial culture is in a dry form. Another embodiment of the present invention relates to the microencapsulated microbial culture as described herein, wherein the microencapsulated microbial culture is freeze dried. Yet another embodiment of the present invention relates to the composition as described herein, wherein the composition is a freeze-dried composition. A still further embodiment of the present invention relates to the microencapsulated microbial culture or the composition as described herein, wherein the microencapsulated microbial culture or composition is in the form of a powder and/or a granulate.

Typically, the storage stability of food products can be extended by formulating the product with low water activity. By controlling the water activity (A_{w}), it is possible to predict and regulate the effect of moisture migration on the product. Therefore, an embodiment of the present invention relates to the microencapsulated microbial culture or the composition as described herein, wherein the water activity (A_{w}) of the microencapsulated microbial culture is in the range from 0.01-0.8, preferably in the range from 0.05-0.4.

fMicrobial cultures may find diverse application across many different consumer sectors. Therefore, an aspect of the present invention relates to a product comprising the microencapsulated microbial culture or the composition as described herein, wherein the product is selected from the group consisting of a feed, a plant health product, a food, a beverage and a pharmaceutical product.

Especially the utilization of the microencapsulated microbial culture in animal feed is a preferred application. Thus, an embodiment of the present invention relates to a product comprising the microencapsulated microbial culture or the composition as described herein, wherein the product is a feed product selected from the group consisting of a feed premix, a feed blend, a pet food and a domestic animal feed.

Another preferred area of application is within products comprising LAB. Therefore, an embodiment of the present invention relates to a product comprising the microencapsulated microbial culture or the composition as described herein, wherein the product is a fermented product.

Probiotic-containing products are also a prioritized field of application. Thus, an embodiment of the present invention relates to a product comprising the microencapsulated microbial culture or the composition as described herein, wherein the product is a dairy product selected from the group consisting of yoghurt, cheese, butter, an inoculated sweet milk and a liquid fermented milk product.

Decreasing yield loss of microbial cultures during freezing/FD downstream processing is of great importance, both in terms of quality but also in terms of process efficiency. Similarly, the ability to retain maximum viability of the microbial cultures during ambient storage conditions has commercial and technical advantages. Thus, the methods described herein has been developed with the aim of achieving both objectives. The method described herein relies on complex coacervation of a set of coacervate components that upon complexation entrap the microbial culture within dense phase droplets. In contrast to other conventional techniques in which the microbial culture is just blended with different matrices, the method described herein provides enhanced protection of the microbial culture both during drying and ambient storage.

Thus, an aspect of the present invention relates to a method for preparing a microencapsulated microbial culture or a composition as described herein, said method comprising the steps of:
i) mixing a microbial culture with a first matrix comprising a first coacervate component, and optionally one or more antioxidants, to form a pre-complex solution, and
ii) mixing the pre-complex solution with a second matrix comprising a second coacervate component, and optionally one or more antioxidants, to form a microencapsulated microbial culture,
wherein said first and second coacervate components are octenyl succinic anhydride (OSA) starch or chitosan and wherein said first and second coacervate components are not the same.

An embodiment of the present invention relates to a method as described herein, wherein said first coacervate component is octenyl succinic anhydride (OSA) starch and said second coacervate component is chitosan.

To obtain a final dry microbial culture product with as high viability over time as possible, it is necessary to carefully select and adjust content of the starting materials. Therefore, an embodiment of the present invention relates to the method as described herein, wherein the microbial culture of step i) is a concentrated microbial culture comprising a dry matter content in the range from 5-80 wt%, preferably 15-25 wt%. Another embodiment of the present invention relates to the method as described herein, wherein the microbial culture of step i) is a concentrated microbial culture comprising a dry matter content of at least 5 wt%, such as at least 10 wt%, such as at least 15 wt%, such as at least 20 wt%.

A further embodiment of the present invention relates to the method as described herein, wherein the microbial culture of step i) is a concentrated microbial culture comprising a content of viable microbes in the range from 10⁹ - 10¹² CFU/g, preferably approximately 10¹¹ CFU/g. A still further embodiment of the present invention relates to the method as described herein, wherein the microbial culture of step i) is a concentrated microbial culture comprising a content of viable microbes of at least 10⁹ CFU/g, such as at least 10¹⁰ CFU/g, preferably at least 10¹¹ CFU/g.

The starting materials are utilized in a complex coacervation process to yield the microencapsulated microbial cultures. Each process step is adjusted to obtain coacervate complexes suitable for the intended final application, with some overall parameters, such as the temperature and duration of mixing, being defined. Thus, an embodiment of the present invention relates to the method as described herein, wherein the microbial culture of step i) is mixed with the first matrix for a time period in the range of 5 min to 6 hours at a temperature in the range of 4°C to 45°C.

Another embodiment of the present invention relates to the method as described herein, wherein the microbial culture of step i) is mixed with the first matrix for a time period in the range of 10-30 min.

Yet another embodiment of the present invention relates to the method as described herein, wherein the microbial culture of step i) is mixed with the first matrix at a temperature in the range of 10°C to 15°C.

A further embodiment of the present invention relates to the method as described herein, wherein mixing of the pre-complex solution with the second matrix in step ii) is carried out for a time period in the range of 5 min to 6 hours at a temperature in the range of 4°C to 45°C.

A still further embodiment of the present invention relates to the method as described herein, wherein mixing of the pre-complex solution with the second matrix in step ii) is carried out for a time period in the range of 5 min to 2 hours, preferably for a time period in the range of 10-20 min.

An even further embodiment of the present invention relates to the method as described herein, wherein mixing of the pre-complex solution with the second matrix in step ii) is carried out at a temperature in the range of 10°C to 15°C.

As explained herein, the pH of the solution during complex formation guides the extent of electrostatic interactions between the coacervate components. Therefore, it is necessary to control the pH throughout the complex coacervation process. Thus, an embodiment of the present invention relates to the method as described herein, wherein the pH in step ii) is adjusted using an acid or a base.

Another embodiment of the present invention relates to the method as described herein, wherein the acid is selected from the group consisting of orthophosphoric acid, hydrochloric acid and acetic acid, and mixtures thereof.

A further embodiment of the present invention relates to the method as described herein, wherein the base is selected from the group consisting of NaOH, KOH and liquid ammonia, and mixtures thereof.

It is to be understood that the skilled person may also select and use other acids and bases suitable for adjusting the pH during the complex coacervation process. Given the relevant pKa values of OSA starch and chitosan, only pH values suitable for facilitating electrostatic interactions between these two coacervation components are applicable. As explained herein, the pH may for instance be adjusted to vary the protonation stage of the amine groups of chitosan. Thus, an embodiment of the present invention relates to the method as described herein, wherein the pH in step ii) is in the range between 6-8, preferably between 6.25-7.5.

As an additive for inclusion in a variety of different end products, microbial cultures, such as LAB, are often supplied as dried powders. The dry form makes the microbial cultures easy to transport, store and handle prior to final processing. Therefore, an embodiment of the present invention relates to the method as described herein further comprising a step iii) subsequent to step ii), wherein step iii) comprises freezing said microencapsulated microbial culture to obtain a frozen microencapsulated microbial culture.

Another embodiment of the present invention relates to the method as described herein further comprising a step iv) subsequent to step iii), wherein step iv) comprises sublimating water from said frozen microencapsulated microbial culture to obtain a dried microencapsulated microbial culture.

A further embodiment of the present invention relates to the method as described herein, wherein step iv) is carried out by a technique selected from the group consisting of spray drying, vacuum drying, air drying, freeze drying, tray drying and vacuum tray drying.

A still further embodiment of the present invention relates to the method as described herein, wherein the technique used in step iv) is freeze drying and wherein said freeze drying is performed at 0.005 to 1 mbar at -45°C to 45°C until complete water removal.

An even further embodiment of the present invention relates to the method as described herein, wherein said freeze drying is performed at 0.1 to 0.4 mbar. Yet another embodiment of the present invention relates to the method as described herein, wherein said freeze drying is performed at at least 0.1 mbar, such as at least 0.2 mbar, such as at least 0.3 mbar, such as at least 0.4 mbar.

Another embodiment of the present invention relates to the method as described herein, wherein said freeze drying is performed at 15°C to 35°C. An additional embodiment of the present invention relates to the method as described herein, wherein said freeze drying is performed at at least 15°C, such as at least 20°C, such as at least 25°C, such as at least 30°C.

Depending on the application of the microencapsulated microbial culture, it may be advantageous to receive the microencapsulated microbial culture in frozen or dried form. Therefore, an embodiment of the present invention relates to the method as described herein, wherein said method further comprises:
(v) packing said frozen microencapsulated microbial culture obtained in step
(iii) or the dried microencapsulated microbial culture obtained in step (iv).

The microencapsulated microbial culture is the result of the complex coacervation process as describer herein. Consequently, an aspect of the present invention relates to a microencapsulated microbial culture or a composition obtainable by a method as described herein.

Preferably, the microencapsulated microbial culture is utilized as additive in the preparation of an end product. Thus, an aspect of the present invention relates to the use of a microencapsulated microbial culture or a composition as described herein in a product selected from the group consisting of a feed, a plant health product, a food, a beverage and a pharmaceutical product.

The listing or discussion of an apparently prior published document in this specification should not necessarily be taken as an acknowledgement that the document is part of the state of the art or is common general knowledge.

Preferences, options and embodiments for a given aspect, feature or parameter of the invention should, unless the context indicates otherwise, be regarded as having been disclosed in combination with any and all preferences, options and embodiments for all other aspects, features and parameters of the invention. This is especially true for the description of the microencapsulated microbial culture and all its features, which may readily be part of the final composition obtained by the method as described herein. Embodiments and features of the present invention are also outlined in the following items.

### Specific embodiments

The present disclosure comprises a microencapsulated microbial culture, said microencapsulated microbial culture comprising:
i) a microbial culture,
ii) a first matrix comprising a first coacervate component, and
iii) a second matrix comprising a second coacervate component,
wherein said first and second coacervate components are octenyl succinic anhydride (OSA) starch or chitosan and wherein said first and second coacervate components are not the same.

The first or second matrix may comprise one or more antioxidants.

The microencapsulated microbial culture may comprise a first coacervate component, which is octenyl succinic anhydride (OSA) starch and said second coacervate component, which is chitosan.

The microencapsulated microbial culture may comprise a microbial culture, which is a bacterium or a yeast.

In one embodiment, the microbial culture is or comprises a lactic acid bacteria (LAB) of a genus selected from the group consisting of *Lactobacillus, Holzapfelia, Amylolactobacillus, Bombilactobacillus, Companilactobacillus, Lapidilactobacillus, Agrilactobacillus, Schleiferilactobacillus, Loigolactobacilus, Lacticaseibacillus, Latilactobacillus, Dellaglioa, Liquorilactobacillus, Ligilactobacillus, Lactiplantibacillus, Furfurilactobacillus, Paucilactobacillus, Limosilactobacillus, Fructilactobacillus, Acetilactobacillus, Apilactobacillus, Levilactobacillus, Secundilactobacillus and Lentilactobacillus, Leuconostoc, Pediococcus, Lactococcus, Streptococcus, Enterococcus, Bifidobacterium, Brevibacterium,* and *Staphylococcus.*

In one embodiment, the microbial culture is or comprises a lactic acid bacteria (LAB) of a genus selected from the group consisting of *Lactobacillus, Limosilactobacillus, Lacticaseibacillus, Ligilactobacillus, Lacticaseibacillus, Lacticaseibacillus, Lactiplantibacillus, Limosilactobacillus, Ligilactobacillus, Lentilactobacillus, Latilactobacillus, Companilactobacillus, Latilactobacillus* and *Lactiplantibacillus.*

In one embodiment, the microbial culture is or comprises of a species of *Limosilactobacillus reuteri, Lacticaseibacillus rhamnosus, Ligilactobacillus salivarius, Lacticaseibacillus casei, Lacticaseibacillus paracasei subsp. paracasei, Lactiplantibacillus plantarum subsp. plantarum, Limosilactobacillus fermentum, Ligilactobacillus animalis, Lentilactobacillus buchneri, Latilactobacillus curvatus, Companilactobacillus futsaii, Latilactobacillus sakei subsp., Lactiplantibacillus pentosus, Lactobacillus acidophillus, Lactobacillus helveticus, Lactobacillus gasseri* and *Lactobacillus delbrueckii.*

In one embodiment, the microbial culture is or comprises *Ligilactobacillus animalis* 51 (LA51) deposited as DSM 33570 at Leibniz Institute DSMZ-German Collection of Microorganisms and Cell cultures Inhoffenstr. 7B 38124 Braunschweig Germany (Deutsche Sammlung von Mikroorganismen und Zellkulturen (DSMZ) 5 GmbH1 Inhoffenstr. 7B, D-38124 Braunschweig, Germany) by Chr. Hansen A/S, Hørsholm, Denmark on 8 July 2020; *Bifidobacterium animalis* subsp. *lactis* deposited as DSM 15954 at DSMZ; *Streptococcus thermophilus* deposited as DSM 15957 at DSMZ; or *Lactococcus lactis* subsp. *lactis* deposited as DSM 21404 at DSMZ.

The microencapsulated microbial culture may comprise a microbial culture, which is a probiotic culture.

The content of OSA starch in the microencapsulated microbial culture composition may be between 20-40 wt%, such as between 25-37 wt%, preferably between 30-35 wt%.

The content of chitosan in the microencapsulated microbial culture composition may be between 1-10 wt%, such as between 2-8 wt%, preferably between 4-6 wt%.

The ratio (wt%/wt%) of OSA starch to chitosan may be between 75:25 to 98:2, preferably between 85:15 to 95:5.

The ratio (wt%/wt%) of OSA starch to chitosan may be 85:15 or 95:5.

The one or more antioxidants may be selected from the group consisting of trisodium citrate, sodium ascorbate, vitamin E and combinations thereof.

In one specific embodiment, the microencapsulated microbial culture is trisodium citrate.

The content of antioxidants in the composition may be between 2-20 wt%, such as between 5-15 wt%, preferably between 8-12 wt%.

The ratio (wt%/wt%) of the first and second matrix combined to microbial culture may be between 0.5-10.

The the ratio (wt%/wt%) of the first and second matrix combined to microbial culture may be 1.

The pH may be in the range between 6-8, preferably between 6.25-7.5.

In one embodiment, the microencapsulated microbial culture further comprising a hydrophobic coating.

The hydrophobic coating may be one or more fats or waxes and mixture thereof.

In one specific embodiment, the microbial culture comprises a content of viable microbes in the range from 10⁶ - 10¹² CFU/g after storage for 4 weeks at 37°C and A_{w} ≤ 0.15.

In one embodiment, a composition comprising the microencapsulated microbial culture according to embodiments of the invention is provided.

The composition may comprise complex coacervates comprising the microbial culture, the first matrix and the second matrix.

The complex coacervates may be arranged with an interior part comprising the microbial culture and the first matrix, and an exterior part comprising the second matrix.

The composition may further comprise one or more additives selected from the group consisting of food-grade ingredients, pharmaceutical ingredients and excipients.

The food-grade ingredients may be selected from the group consisting of lactose, maltodextrin, whey protein, casein, corn starch, dietary fibres, gums and gelatine.

The pharmaceutical ingredients may be selected from the group consisting of calcium carbonate, sodium carboxymethyl cellulose, talc, polydimethylsiloxane, hydroxypropyl cellulose and hydroxypropyl methylcellulose.

The excipients may be selected from the group consisting of microcrystalline cellulose, titanium dioxide and aluminium silicate.

The composition may be a freeze-dried composition.

In one embodiment, the microencapsulated microbial culture according to embodiments of the invention or the composition according to embodiments of the invention is in the form of a powder and/or a granulate.

The water activity (A_{w}) of the microencapsulated microbial culture may be in the range from 0.01- 0.8, preferably in the range from 0.05-0.4.

In one embodiment, a product selected from the group consisting of a feed, a plant health product, a food, a beverage and a pharmaceutical product is provided, said product comprising the microencapsulated microbial culture according embodiments of the invention or the composition according to embodimetns of the invention.

In one embodiment, a method for preparing a microencapsulated microbial culture according to embodiments of the invention or a composition according to embodiments of the invention is provided, said method comprising the steps of:
i) mixing a microbial culture with a first matrix comprising a first coacervate component and optionally one or more antioxidants to form a pre-complex solution, and
ii) mixing the pre-complex solution with a second matrix comprising a second coacervate component and optionally one or more antioxidants to form a microencapsulated microbial culture,
wherein said first and second coacervate components are octenyl succinic anhydride (OSA) starch or chitosan and wherein said first and second coacervate components are not the same.

Said first coacervate component may be octenyl succinic anhydride (OSA) starch and said second coacervate component may be chitosan.

The microbial culture of step i) above may be a concentrated microbial culture comprising a dry matter content in the range from 5-80 wt%, preferably 15-25 wt%.

The microbial culture of step i) above may be a concentrated microbial culture comprising a content of viable microbes in the range from 10⁹ - 10¹² CFU/g, preferably approximately 10¹¹ CFU/g.

The microbial culture of step i) above may be mixed with the first matrix for a time period in the range of 5 min to 6 hours at a temperature in the range of 4°C to 45°C.

The microbial culture of step i) above may be mixed with the first matrix for a time period in the range of 10-30 min.

The microbial culture of step i) above may be mixed with the first matrix at a temperature in the range of 10°C to 15°C.

Mixing of the pre-complex solution with the second matrix in step ii) above, may be carried out for a time period in the range of 5 min to 6 hours at a temperature in the range of 4°C to 45°C.

Mixing of the pre- complex solution with the second matrix in step ii) above, may be carried out for a time period in the range of 5 min to 2 hours, preferably for a time period in the range of 10-20 min.

Mixing of the pre- complex solution with the second matrix in step ii) above, may be carried out at a temperature in the range of 10°C to 15°C.

The pH in step ii) above, may be adjusted using an acid or a base.

The acid may be selected from the group consisting of orthophosphoric acid, hydrochloric acid and acetic acid, and mixtures thereof.

The base may be selected from the group consisting of NaOH, KOH and liquid ammonia, and mixtures thereof.

The pH in step ii) above may be in the range between 6-8, preferably between 6.25-7.5.

In one embodiment, the method above further comprising a step iii) subsequent to step ii), wherein step iii) comprises freezing said microencapsulated microbial culture to obtain a frozen microencapsulated microbial culture.

In one embodiment, the method above further comprises a step iv) subsequent to step iii), wherein step iv) comprises sublimating water from said frozen microencapsulated microbial culture to obtain a dried microencapsulated microbial culture.

In one embodiment, step iv) is carried out by a technique selected from the group consisting of spray drying, vacuum drying, air drying, freeze drying, tray drying and vacuum tray drying.

In one specific embodiment, the drying is freeze drying and said freeze drying is performed at 0.005 to 1 mbar at - 45°C to 45°C until complete water removal.

In one preferred embodiment, said freeze drying is performed at 0.1 to 0.4 mbar.

In one preferred embodiment, said freeze drying is performed at 15°C to 35°C.

The method may further comprise:
(v) packing said frozen microencapsulated microbial culture obtained in step (iii) or the dried microencapsulated microbial culture obtained in step (iv).

In one embodiment, a microencapsulated microbial culture or a composition obtainable by a method according to embodiments of the invention is provided.

In one embodiment, use of a microencapsulated microbial culture or a composition according embodiments of the invention, in a product selected from the group consisting of a feed, a plant health product, a food, a beverage and a pharmaceutical product, is provided.

The invention will now be described in further details in the following non-limiting examples.

### EXAMPLES

### EXAMPLE 1: COMPLEX FORMATION WITH COACERVATION COMPONENTS

### Materials and methods

The HiCap^{®} 100 (octenyl succinic anhydride) starch was supplied by Ingredion, Germany, GmbH. Chitosan from crab shell was procured from Meron group, Cochin, India. Tri-sodium citrate dihydrate was procured from Rankem, India. Maltodextrin and trehalose were used from Roquette, France. Glacial acetic acid was supplied by Thomas baker, India. NaOH (10%, w/w) solution was prepared by dissolving 10 g of NaOH pellets in 90 g of Water.

### Preparation of OSA starch (HiCap^{®}100) and chitosan complexes

The OSA starch solution was prepared by dispersing 0.1 g of HiCap^{®}100 OSA starch powder in 99.9 g of deionized water to get final solution with a concentration of 0.1 wt%. Similarly, the chitosan solution was prepared by dispersing 0.1 g of chitosan in 99.9 g of 0.5 wt% acetic acid solution and both the solution were allowed to hydrate overnight on a magnetic stirrer at 400 rpm at a temperature of 25°C. Before starting the complexation experiment, the pH of the 0.1 wt% chitosan dispersion was adjusted to pH 5 using 0.1M acetic acid. The HiCap^{®}100 OSA starch: chitosan complex formation was studied at different coacervate component ratios of HiCap^{®}100 OSA starch: chitosan (25:75, 50:50, 75:25, 85:15, 95:5) and the total coacervate component concentration was kept constant to 0.1 wt%. Each of the solutions was weighed separately to get the desired concentration and mixed together in a glass beaker under agitation on a magnetic stirrer at 25°C. The electrostatic interaction between HiCap^{®}100 OSA starch and chitosan was studied by varying pH from 5 to 8 where the pH of the reaction mixture was adjusted using 0.1 M NaOH solution. As the pH of reaction mixture was changed, small amount of sample was withdrawn at predetermined pH intervals of 0.25 and the optical density (OD) of the reaction mixture was measure at 600 nm using a UV-vis spectrophotometer (Thermo Fisher Scientific, MA, USA). The same sample was also used to check interaction and macroscopic structure formation using a phase contrast microscope at magnification of 40X. The data was then collected to plot pH versus OD to find out the maximum insoluble complex formation between HiCap^{®}100 starch and chitosan.

### Preparation of Matrices for encapsulation

### - Preparation of solutions for HiCap^{®}100 OSA starch and chitosan complexes

In all the complex coacervation experiments, there are two matrices involved which are needed and they were prepared as follows,

### - First matrix for HiCap^{®}100 OSA starch:chitosan complex with coacervate component ratio 85:15

1.10 g of HiCap^{®}100 OSA starch and 0.30 g of tri-sodium citrate dihydrate were weighed and added to a 50 ml centrifuge tube. 13.30 g of water was added to the same tube and the ingredients were allowed to dissolve into the water on a Revolver/Rotator (ThermoFisher Scientific, MA, USA) at a speed of 25 rpm. After dissolving the ingredients, the pH of the solution was adjusted to 7.25 using 10% NaOH solution.

### - Second matrix for HiCap^{®}100 OSA starch:chitosan complex with coacervate component ratio 85:15

0.18 g of chitosan was weighed and dispersed into 18.06 g of a 0.5 wt% acetic acid solution. The solution was allowed to hydrate overnight with stirring. After the chitosan was completely hydrated, the pH of the chitosan dispersion was adjusted slowly to pH of 7.25 using 10 wt% NaOH solution.

### - First matrix for HiCap^{®}100 OSA starch:chitosan complex with coacervate component ratio 95:5

1.66 g of HiCap^{®}100 OSA starch and 0.44 g of tri-sodium citrate dihydrate were weighed and added to a 50 ml centrifuge tube. 19.37 g of water was added to the same tube and the ingredients were allowed to dissolve into the water on a Revolver/Rotator at a speed of 25 rpm. After dissolving the ingredients, the pH of the solution was adjusted to 6.50 using 10% NaOH solution.

### - Second matrix for HiCap^{®}100 OSA starch:chitosan complex with coacervate component ratio 95:5

0.08 g of chitosan was weighed and dispersed into 8.17 g of a 0.5 wt% acetic acid solution. The solution was allowed to hydrate overnight with intermittent stirring. After the chitosan was completely hydrated, pH of the chitosan dispersion was adjusted slowly to pH of 6.50 using 10 wt% NaOH solution.

### - Chitosan (Control) experiment

0.25 g of chitosan and 0.75 g of tri-sodium citrate dihydrate were weighed and added to a 50 ml centrifuge tube. 26.50 g of a 0.5 wt% acetic acid solution was added to the same tube and allowed to hydrate overnight with stirring. After the chitosan was completely hydrated, pH of the chitosan dispersion was adjusted slowly to pH of 6.50 using 10 wt% NaOH solution.

### - Heat sterilization of matrix solutions:

All the solutions were heat sterilized before they were used in the formulations concentrate. Tri-sodium citrate dihydrate solution was filtered through a 0.22 micron syringe filter. The first matrix containing HiCap^{®}100 OSA starch and other ingredients were sterilized at 75°C for 20 min and the second matrix containing chitosan was heat sterilized at 90°C for 20 mins and then cooled down to room temperature before use.

### - Production of microbial cultures

The *Bifidobacterium animalis lactis* (BB-12^{®}, DSM 15954) of the present invention was inoculated into a De Man, Rogosa and Sharpe (MRS) liquid medium (BD DifcoTM Lactobacilli MRS Agar, Fisher Scientific) supplemented with 0.5g/L of L-cysteine hydrochloride (Sigma-Aldrich, Inc.) and cultured anaerobically at 37°C. for 24 hours. After the 24 hours of growth, cell in the medium were concentrated by 25 fold using centrifugation. These concentrated cells were used in microencapsulation of complex coacervates experiments throughout the invention. Similar to this, *Ligilactobacillus animalis* LA51 (DSM 33570), *Streptococcus thermophilus* TH4 (DSM 15957)and *Lactococcus lactis* R607 (DSM 21404) were grown in De Man, Rogosa and Sharpe (MRS) liquid medium anaerobically at 37°C. for 24 hours. These concentrated cells were used in microencapsulation of complex coacervates experiments throughout the invention.

### Encapsulation of bacteria

### - Encapsulation of bacteria using complex coacervation

The cell concentrate was weighed in a 50 ml falcon tube and the first matrix was added to the same tube. The first matrix and cell concentrate were mixed for 15 min at less than 10°C on a Revolver/Rotator (25 rpm). Subsequently, the second matrix was added to the falcon tube. Cell concentrate and matrix 1 and 2 were mixed for 15 min at at least 10°C on a Revolver/Rotator (25 rpm). After the incubation time, the mixture was filled into a sterile pipette. The mixture of cell concentrate and matrix 1 and 2 were dropped into liquid nitrogen to form pre-freeze dried pellets (PFDs). These PFDs were kept at -70 °C until freeze drying.

### - Freeze drying of the samples

The PFD's of individual samples were loaded into a labelled pre-frozen metal container and the containers were transferred to a freeze dryer. The PFDs were dried using a Martin Christ freeze dryer (Germany, GmbH) for 26 h. Subsequently, the freeze dried (FD) pellets were filled in 50 mL closed falcon tubes and stored at - 70°C until further analysis.

### - Preparation of samples for stability study

The FD pellets of each samples were weighed and heat sealed in Aluminium-bags. The Aluminium-bags containing samples were subjected to a storage stability study at accelerated conditions (Temperature = 37°C and A_{w} = 0.05). The samples were withdrawn at pre-determined time intervals and analysed for CFU and A_{w}.

### - Microscopy study of samples:

The microscopic images of complex coacervates prepared by interaction of coacervate component mixtures and individuals were taken on 40X magnification. All the complex coacervates containing cells were taken under 100X magnification using a Nikon microscope (Tokyo, Japan).

### - Complex formation with coacervation components

The interaction between the two coacervate components, HiCap^{®}100 OSA starch and chitosan, was investigated to determine suitable pH ranges and ratios of coacervate components for efficient complex formation.

### Methods

The complex coacervates of HiCap^{®}100 OSA starch:chitosan were prepared at ratios of 85:15 and 95:5 according to the materials and methods part presented above.

### Results

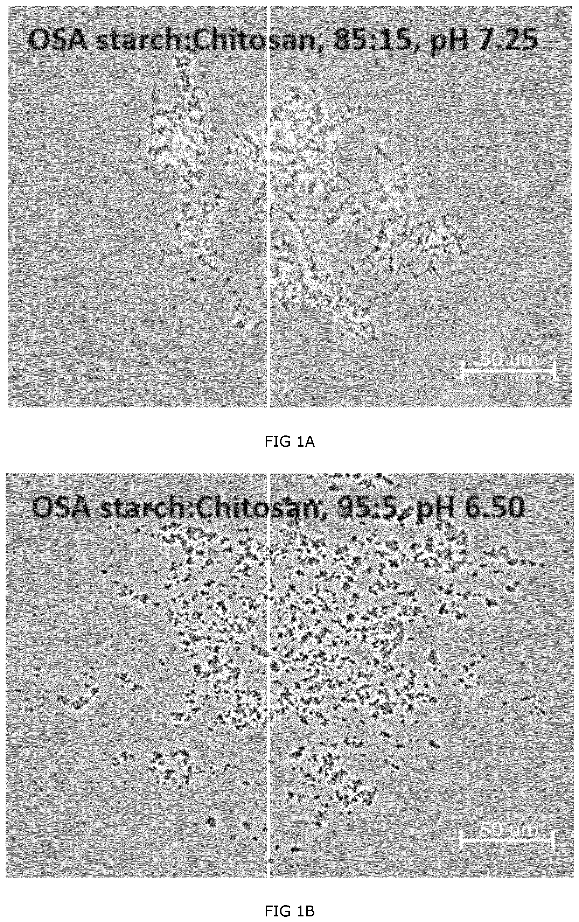

It was found that complex formation was favoured for formulations comprising high contents of HiCap^{®}100 OSA starch, and especially in the pH range of 6 to 8. Under these conditions, formation of insoluble complex coacervates could be visualized as exemplified by complex coacervates of HiCap^{®}100 OSA starch:chitosan at ratios of 85:15 (Figure 1A) and 95:5 (Figure 1B), at pH 7.25 and 6.5, respectively.

### Conclusion

This example demonstrates that OSA starch and chitosan form complex coacervates for a range of pH values and coacervate component ratios.

### EXAMPLE 2: MICROENCAPSULATION OF MICROBIAL CULTURES IN COMPLEX COACERVATES

Microencapsulation of microbial cultures were demonstrated by incorporation of Ligilactobacillus animalis 51 (LA51) or *Bifidobacterium lactis* (BB-12^{®}) or S. *thermophilus* (TH4) or *Lactococcus lactis* R607 in complex coacervates of HiCap^{®}100 OSA starch and chitosan and the protective effect of the microencapsulation upon freeze drying were evaluated.

### Methods

The two complex coacervates of Example 1 (ratios 85:15 and 95:5) were used as a starting point for formulating LA51, BB-12^{®}, TH4 or *Lactococcus lactis* (R607) as microencapsulated cultures. Each complex coacervate was formulated with an antioxidant (herein trisodium citrate) and *Ligilactobacillus animalis* 51 (LA51) or *Bifidobacterium lactis* (BB-12^{®}) or *Streptococcus thermophilus* TH4 or *Lactococcus lactis* (R607) in a procedure that involved addition of the coacervate components in sequential steps.

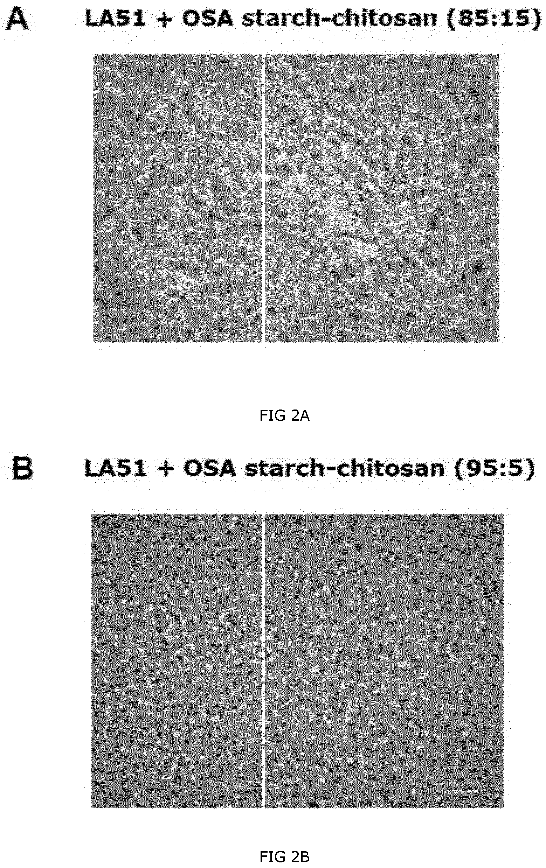

In a first step, frozen biomass of *Ligilactobacillus animalis* 51 (LA51) or *Bifidobacterium lactis* (BB12) or S. *thermophilus* (TH4) or *Lactococcus lactis* (R607)was added to a first matrix containing HiCap^{®}100 OSA starch and trisodium citrate and mixed at 10 °C until the frozen biomass was liquified in the matrix using a tube revolver/rotator (ThermoFisher Scientific). In a second step, a second matrix containing chitosan was added to the predetermined ratio of HiCap^{®}100 OSA starch to chitosan (here 85:15 and 95:5) and the pH was adjusted by addition of 1M NaOH solution. The sample with an HiCap^{®}100 OSA starch to chitosan ratio of 85:15 (referred to as 'sample 1') was adjusted to pH 7.25 and the sample with an HiCap^{®}100 OSA starch to chitosan ratio of 95:5 (referred to as 'sample 2') was adjusted to pH 6.50. Following adjustment of pH, the complex formation reaction was carried out for 10 min at 10°C using the tube revolver/rotator. The ratio (wt%/wt%) of LA51 to the first a second matrix combined was 1. Exemplary microscope images of sample 1 (figure 2A) and 2 (figure 2B) are provided to illustrate the morphology of the complex coacervates of LA51. This is also disclosed in the materials and methods part above.

**Table 2. Composition of final freeze dried granulated samples.**

| ***Ingredient*** | ***Sample 1 (85:15) (wt%*/*wt%)*** | ***Sample 2 (95:5) (wt%*/*wt%)*** |
|---|---|---|
| HiCap^{®}100 OSA starch | 33.48 | 37.42 |
| Chitosan | 5.91 | 1.97 |
| Trisodium citrate or Sodium ascorbate | 10.13 | 10.13 |
| Bacteria | 49.49 | 49.49 |
| Moisture | 0.97 | 0.97 |

As a reference point, samples of *Ligilactobacillus animalis* 51 (LA51) or *Bifidobacterium lactis* (BB12) or S. *thermophilus* (TH4) or *Lactococcus lactis* (R607)comprising (i) a reference cryoprotectant (trehalose, maltodextrin and trisodium citrate) (referre to as 'reference cryoprotectant') or (ii) only chitosan and trisodium citrate (referred to as 'control sample') were prepared. The control sample was prepared with 5 wt% trisodium citrate and the pH being adjusted to 6.50.

The resulting formulations containing *Ligilactobacillus animalis* 51 (LA51) or *Bifidobacterium lactis* (BB12) or S. *thermophilus* (TH4) or *Lactococcus lactis* (R607)were filled in a sterile pipette and added dropwise into liquid nitrogen to form pellets (referred to as 'PFD') and then stored at -80°C prior to freeze drying. The PFDs were dried using a freeze drier (Martin Christ, GmbH) using a safe profile (0.3 mbar, 32°C for a time of 26 hours). After freeze drying, freeze dried granulates (referred to as 'FD granulates') were tested for water activity (A_{w}) and colony forming unit (CFU/g). CFU/g was determined as described herein under the definition of viability.

### Results

Samples 1 (6.88%) and 2 (41.30%) both showed better protection against freeze drying compared to the reference cryoprotectant as measured by the count of CFU/g (figure 2). Least effective was the control sample, wherein only chitosan and trisodium citrate were added to LA51.

### Conclusion

The example demonstrates that microencapsulation of microbial cultures in complex coacervates of OSA starch and chitosan can enhance protection of the microbial culture against freeze drying and thereby decrease the loss in viability.

### EXAMPLE 3: STORAGE STABILITY OF MICROENCAPSULATED MICROBIAL CULTURES

Stability of microencapsulated microbial cultures was investigated to evaluate the effect on culture viability over time when exposed to environmental stress conditions.

### Methods

The FD granulates of samples 1 and 2 were weighed and heat sealed in Aluminium-bags. The Aluminium-bags containing samples were subjected to storage stability study at accelerated conditions (Temperature = 37°C and A_{w} = 0.05). The samples were withdrawn at pre-determined time intervals and analysed for CFU/g and A_{w}.

Storage stability was determined as described herein under the definition of storage stability.

### Results

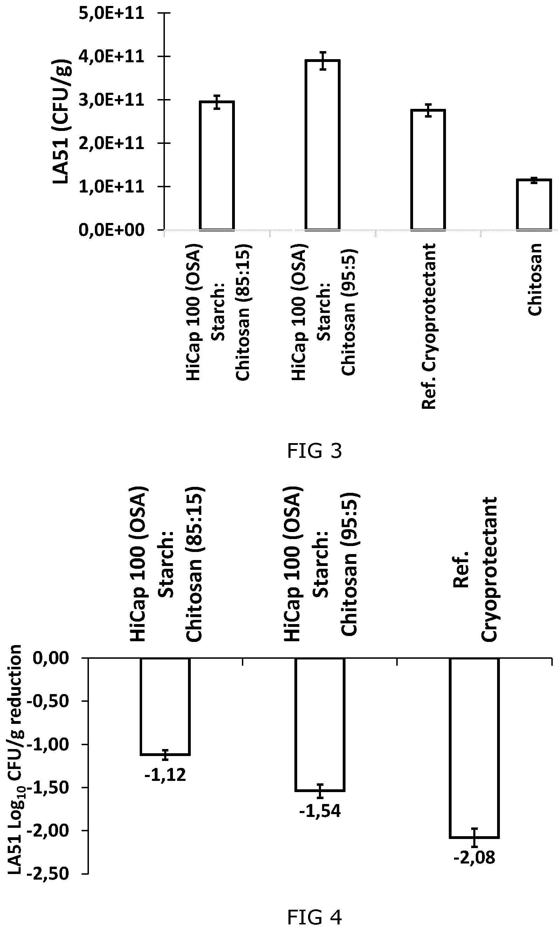

Both complex coacervation samples showed significantly increased storage stability compared to the reference cryoprotectant. Thus, the reduction in viability of the reference cryoprotectant was 2.08 log units compared to 1.54 (sample 2) and 1.12 (sample 1) log units of the complex coacervation samples (Figure 4).

### Conclusion

The example demonstrates that the OSA starch:chitosan complex coacervation process efficiently combines the functional properties of OSA starch and chitosan to yield a protective microencapsulation that increases stability of microbial cultures stored under accelerated storage conditions.

### EXAMPLE 4: FURTHER STORAGE STABILITY OF MICROENCAPSULATED MICROBIAL CULTURES

Stability of microencapsulated microbial cultures was investigated to evaluate the effect on culture viability over time when exposed to environmental stress conditions.

### Methods

The FD granulates of samples 1 and 2 were weighed and heat sealed in Aluminium-bags. The Aluminium-bags containing samples were subjected to storage stability study at accelerated conditions (Temperature = 37°C and a_{w} = 0.05). The samples were withdrawn at pre-determined time intervals and analysed for CFU/g and a_{w}.

Storage stability was determined as described herein under the definition of storage stability.

### Results

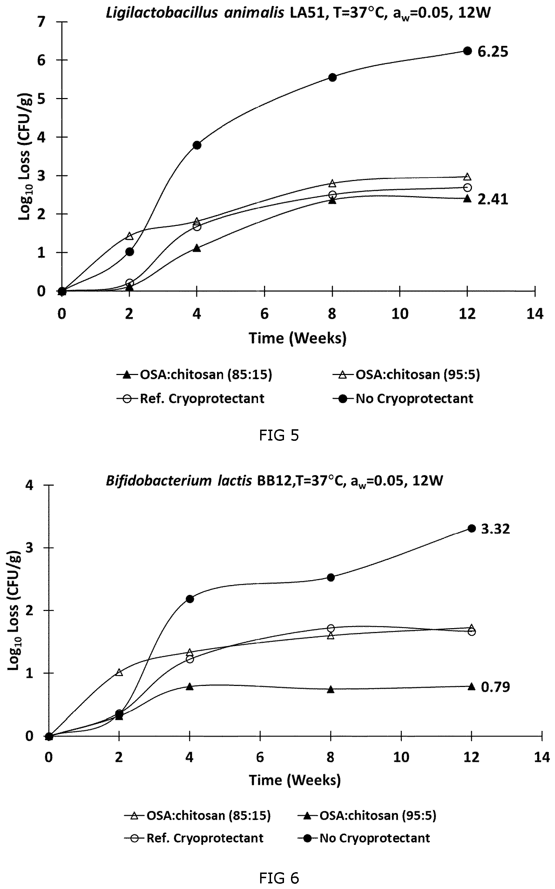

Figure 5 shows the reduction in viability of freeze-dried granulates of *Ligilactobacillus animalis* 51 (LA51) encapsulated in either octenyl succinic anhydride (OSA) starch-chitosan complex coacervates (ratios 85:15 or 95:5) or the reference cryoprotectant or No cryoprotectant, upon storage in 12 weeks at a_{w} ≤ 0.05, T=37°C.

The FD granulates of *Ligilactobacillus animalis* 51 (LA51) encapsulated octenyl succinic anhydride (OSA) starch-chitosan complexes (OSA:chitosan, 85:15) showed significantly higher protection compared to Ref. cryoprotectant, in which there was 2.41 Log₁₀ Loss (CFU/g) when stored at accelerated storage conditions i.e. a_{w} ≤ 0.05, T=37°C after 12 W (Fig. 5). In complexation process the LA51 gets encapsulated/ entrapped between two different biopolymers, whereas in conventional technique which is just the blending of LA51 with the different matrices which doesn't provide the complete protection to bacterial cells. The OSA starch-Chitosan complex coacervation process which allowed to combine the functional properties of OSA starch and chitosan together resulted in giving the novel functional ingredient combination (formulation composition) which not only provided cryo/lyo protection but also provided a better stability to LA51 under accelerated storage conditions compared to Ref. cryoprotectant.

Figure 6 shows the reduction in viability of freeze-dried granulates of *Bifidobacterium lactis* (BB-12^{®}) encapsulated in either octenyl succinic anhydride (OSA) starch-chitosan complex coacervates (ratios 85:15 or 95:5) or the reference cryoprotectant or No cryoprotectant, upon storage in 12 weeks at a_{w} ≤ 0.05, T=37°C.

The FD granulates of *Bifidobacterium lactis* (BB-12^{®}) encapsulated octenyl succinic anhydride (OSA) starch-chitosan complexes (OSA:chitosan, 95:5) showed significantly higher protection compared to Ref. cryoprotectant and No cryoprotectant, in which there was 0.79 Log₁₀ Loss (CFU/g) when stored at accelerated storage conditions i.e. a_{w} ≤ 0.05, T=37°C after 12 W (Fig. 6). In complexation process the BB-12^{®} gets encapsulated/ entrapped between two different biopolymers, whereas in conventional technique which is just the blending of BB-12^{®} with the different matrices which doesn't provide the complete protection to bacterial cells. The OSA starch-chitosan complex with 95:5 ratio and optimal interaction pH of 6.5 has better compatibility with BB-12^{®} and thus formed an intact structure binding to the cell to enhance the protection accelerated conditions. The OSA starch-Chitosan complex coacervation process which allowed to combine the functional properties of OSA starch and chitosan together resulted in giving the novel functional ingredient combination (formulation composition) which not only provided cryo/lyo protection but also provided a better stability to BB-12^{®} under accelerated storage conditions compared to Ref. cryoprotectant.

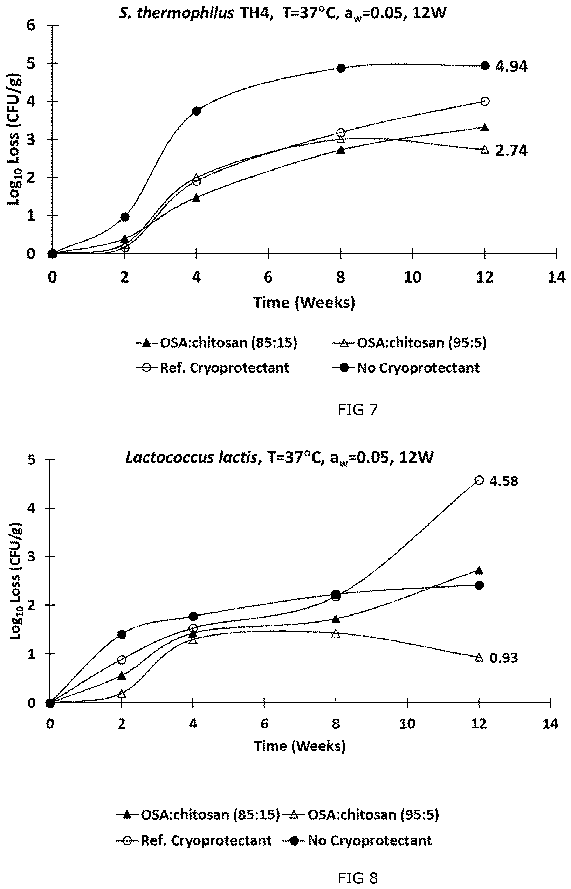

Figure 7 shows the reduction in viability of freeze-dried granulates of S. *thermophilus* (TH4) encapsulated in either octenyl succinic anhydride (OSA) starch-chitosan complex coacervates (ratios 85:15 or 95:5) or the reference cryoprotectant or No cryoprotectant, upon storage in 12 weeks at a_{w} ≤ 0.05, T=37°C.

The FD granulates of S. *thermophilus* (TH4) encapsulated octenyl succinic anhydride (OSA) starch-chitosan complexes (OSA:chitosan, 95:5) showed significantly higher protection compared to Ref. cryoprotectant and No cryoprotectant, in which there was 2.74 Log₁₀ Loss (CFU/g) when stored at accelerated storage conditions i.e. a_{w} ≤ 0.05, T=37°C after 12 W (Fig. 7). In complexation process the TH4 gets encapsulated/ entrapped between two different biopolymers, whereas in conventional technique which is just the blending of TH4 with the different matrices which doesn't provide the complete protection to bacterial cells. The OSA starch-chitosan complex with 95:5 ratio and optimal interaction pH of 6.5 has better compatibility with TH4 and thus formed an intact structure binding to the cell to enhance the protection accelerated conditions. The OSA starch-Chitosan complex coacervation process which allowed to combine the functional properties of OSA starch and chitosan together resulted in giving the novel functional ingredient combination (formulation composition) which not only provided cryo/lyo protection but also provided a better stability to TH4 under accelerated storage conditions compared to Ref. cryoprotectant.

Figure 8 shows the reduction in viability of freeze-dried granulates of *Lactococcus lactis* (R607) encapsulated in either octenyl succinic anhydride (OSA) starch-chitosan complex coacervates (ratios 85:15 or 95:5) or the reference cryoprotectant or No cryoprotectant, upon storage in 12 weeks at a_{w} ≤ 0.05, T=37°C.

The FD granulates of *Lactococcus lactis* (R607) encapsulated octenyl succinic anhydride (OSA) starch-chitosan complexes (OSA:chitosan, 95:5) showed significantly higher protection compared to Ref. cryoprotectant and No cryoprotectant, in which there was 0.93 Log₁₀ Loss (CFU/g) when stored at accelerated storage conditions i.e. a_{w} ≤ 0.05, T=37°C after 12 W (Fig. 8). In complexation process the *Lactococcus lactis* (R607) gets encapsulated/ entrapped between two different biopolymers, whereas in conventional technique which is just the blending of *Lactococcus lactis* (R607) with the different matrices which doesn't provide the complete protection to bacterial cells. The OSA starch-chitosan complex with 95:5 ratio and optimal interaction pH of 6.5 has better compatibility with TH4 and thus formed an intact structure binding to the cell to enhance the protection accelerated conditions. The OSA starch-Chitosan complex coacervation process which allowed to combine the functional properties of OSA starch and chitosan together resulted in giving the novel functional ingredient combination (formulation composition) which not only provided cryo/lyo protection but also provided a better stability to *Lactococcus lactis* (R607) under accelerated storage conditions compared to Ref. cryoprotectant.

### Conclusion

The example demonstrates that the OSA starch: chitosan complex coacervation process efficiently combines the functional properties of OSA starch and chitosan to yield a protective microencapsulation that increases stability of microbial cultures stored under accelerated storage conditions.

### REFERENCES

- Seifert & Mogensen (2002), Bulletin of the IDF, 377, 10-19
- Zheng et al. (2020), Int. J. Syst. Evol. Microbiol., 70, 2782-2858

## Claims

1. A microencapsulated microbial culture, said microencapsulated microbial culture comprising:
i) a microbial culture,
ii) a first matrix comprising a first coacervate component, and
iii) a second matrix comprising a second coacervate component,
wherein said first and second coacervate components are octenyl succinic anhydride (OSA) starch or chitosan and wherein said first and second coacervate components are not the same.

2. The microencapsulated microbial culture according to claim 1, wherein the microbial culture is a bacterium or a yeast.

3. The microencapsulated microbial culture according to any one of claims 1 or 2, wherein the microbial culture is or comprises a lactic acid bacteria (LAB) of a genus selected from the group consisting of *Lactobacillus, Holzapfelia, Amylolactobacillus, Bifidobacterium, Bombilactobacillus, Companilactobacillus, Lapidilactobacillus, Agrilactobacillus, Schleiferilactobacillus, Loigolactobacilus, Lacticaseibacillus, Latilactobacillus, Dellaglioa, Liquorilactobacillus, Ligilactobacillus, Lactiplantibacillus, Furfurilactobacillus, Paucilactobacillus, Limosilactobacillus, Fructilactobacillus, Acetilactobacillus, Apilactobacillus, Levilactobacillus, Secundilactobacillus, Lentilactobacillus, Leuconostoc, Pediococcus, Lactococcus, Streptococcus, Enterococcus, Bifidobacterium, Brevibacterium,* and *Staphylococcus.*

4. The microencapsulated microbial culture according to any one of the preceding claims, wherein the microbial culture is a probiotic culture.

5. The microencapsulated microbial culture according to any one of the preceding claims, wherein the ratio (wt%/wt%) of OSA starch to chitosan is between 75:25 to 98:2, preferably between 85:15 to 95:5.

6. The microencapsulated microbial culture according to any one of the preceding claims, wherein the ratio (wt%/wt%) of the first and second matrix combined to microbial culture is between 0.5-10.

7. The microencapsulated microbial culture according to any one of the preceding claims, wherein the first or second matrix further comprises an antioxidant.

8. The microencapsulated microbial culture according to claim 7, wherein the antioxidant is trisodium citrate, vitamin C, vitamin E, glutathione, or derivatives thereof.

9. A composition comprising the microencapsulated microbial culture according to any one of the preceding claims, wherein the composition further comprises one or more additives selected from the group consisting of food-grade ingredients, pharmaceutical ingredients and excipients.

10. A product comprising the microencapsulated microbial culture according to any one of claims 1-8 or the composition according to claim 9, wherein the product is selected from the group consisting of a feed, a plant health product, a food, a beverage and a pharmaceutical product.

11. A method for preparing a microencapsulated microbial culture according to any one of claims 1-8 or a composition according to claim 9, said method comprising the steps of:
i) mixing a microbial culture with a first matrix comprising a first coacervate component, and optionally one or more antioxidants, to form a pre-complex solution, and
ii) mixing the pre-complex solution with a second matrix comprising a second coacervate component, and optionally one or more antioxidants, to form a microencapsulated microbial culture,
wherein said first and second coacervate components are octenyl succinic anhydride (OSA) starch or chitosan and wherein said first and second coacervate components are not the same.

12. The method according to claim 11, wherein the microbial culture of step i) is mixed with the first matrix for a time period in the range of 5 min to 6 hours at a temperature in the range of 4°C to 45°C, and/or wherein mixing of the pre-complex solution with the second matrix in step ii) is carried out for a time period in the range of 5 min to 6 hours at a temperature in the range of 4°C to 45°C.

13. The method according to any one of claims 11 or 12, wherein the pH in step ii) is in the range between 6-8, preferably between 6.25-7.5.

14. A microencapsulated microbial culture or a composition obtainable by a method according to any one of claims 11-13.

15. Use of a microencapsulated microbial culture or a composition according to any one of claims 1-9 or 14 in a product selected from the group consisting of a feed, a plant health product, a food, a beverage and a pharmaceutical product.

## Patentansprüche

1. Mikroverkapselte mikrobielle Kultur, wobei die mikroverkapselte mikrobielle Kultur Folgendes umfasst:
i) eine mikrobielle Kultur,
ii) eine erste Matrix, die eine erste Koazervatkomponente umfasst, und
iii) eine zweite Matrix, die eine zweite Koazervatkomponente umfasst,
wobei es sich bei der ersten und zweiten Koazervatkomponente um Octenylbernsteinsäureanhydrid(OSA)-Stärke oder Chitosan handelt und wobei die erste und zweite Koazervatkomponente nicht die gleichen sind.

2. Mikroverkapselte mikrobielle Kultur nach Anspruch 1, wobei es sich bei der mikrobiellen Kultur um eine Bakterie oder eine Hefe handelt.

3. Die mikroverkapselte mikrobielle Kultur nach einem der Ansprüche 1 oder 2, wobei die mikrobielle Kultur eine Milchsäurebakterie (LAB) einer Gattung ist oder umfasst, ausgewählt aus der Gruppe, bestehend aus *Lactobacillus, Holzapfelia, Amylolactobacillus, Bifidobacterium, Bombilactobacillus, Companilactobacillus, Lapidilactobacillus, Agrilactobacillus, Schleiferilactobacillus, Loigolactobacilus, Lacticaseibacillus, Latilactobacillus, Dellaglioa, Liquorilactobacillus, Ligilactobacillus, Lactiplantibacillus, Furfurilactobacillus, Paucilactobacillus, Limosilactobacillus, Fructilactobacillus, Acetilactobacillus, Apilactobacillus, Levilactobacillus, Secundilactobacillus, Lentilactobacillus, Leuconostoc, Pediococcus, Lactococcus, Streptococcus, Enterococcus, Bifidobacterium, Brevibacterium* und *Staphylococcus.*

4. Mikroverkapselte mikrobielle Kultur nach einem der vorhergehenden Ansprüche, wobei es sich bei der mikrobiellen Kultur um eine probiotische Kultur handelt.

5. Mikroverkapselte mikrobielle Kultur nach einem der vorhergehenden Ansprüche, wobei das Verhältnis (Gew.-%/Gew.-%) von OSA-Stärke zu Chitosan zwischen 75:25 und 98:2, vorzugsweise zwischen 85:15 und 95:5, liegt.

6. Mikroverkapselte mikrobielle Kultur nach einem der vorhergehenden Ansprüche, wobei das Verhältnis (Gew.-%/Gew.-%) der kombinierten ersten und zweiten Matrix zu mikrobieller Kultur zwischen 0,5 und 10 liegt.

7. Mikroverkapselte mikrobielle Kultur nach einem der vorhergehenden Ansprüche, wobei die erste oder zweite Matrix ferner ein Antioxidans umfasst.

8. Mikroverkapselte mikrobielle Kultur nach Anspruch 7, wobei es sich bei dem Antioxidans um Trinatriumcitrat, Vitamin C, Vitamin E, Glutathion oder Derivate davon handelt.

9. Zusammensetzung, umfassend die mikroverkapselte mikrobielle Kultur nach einem der vorhergehenden Ansprüche, wobei die Zusammensetzung ferner einen oder mehrere Zusätze umfasst, ausgewählt aus der Gruppe, bestehend aus lebensmitteltauglichen Inhaltsstoffen, pharmazeutischen Inhaltsstoffen und Hilfsstoffen.

10. Produkt, umfassend die mikroverkapselte mikrobielle Kultur nach einem der Ansprüche 1 bis 8 oder die Zusammensetzung nach Anspruch 9, wobei das Produkt ausgewählt ist aus der Gruppe, bestehend aus einem Futtermittel, einem Pflanzengesundheitsprodukt, einem Lebensmittel, einem Getränk und einem pharmazeutischen Produkt.

11. Verfahren zum Herstellen einer mikroverkapselten mikrobiellen Kultur nach einem der Ansprüche 1 bis 8 oder einer Zusammensetzung nach Anspruch 9, wobei das Verfahren die folgenden Schritte umfasst:
i) Mischen einer mikrobiellen Kultur mit einer ersten Matrix, die eine erste Koazervatkomponente und gegebenenfalls ein oder mehrere Antioxidantien umfasst, unter Bilden einer Präkomplexlösung und
ii) Mischen der Präkomplexlösung mit einer zweiten Matrix, die eine zweite Koazervatkomponente und gegebenenfalls ein oder mehrere Antioxidantien umfasst, unter Bilden einer mikroverkapselten mikrobiellen Kultur,
wobei es sich bei der ersten und zweiten Koazervatkomponente um Octenylbernsteinsäureanhydrid(OSA)-Stärke oder Chitosan handelt und wobei die erste und zweite Koazervatkomponente nicht die gleichen sind.

12. Verfahren nach Anspruch 11, wobei die mikrobielle Kultur von Schritt i) mit der ersten Matrix über einen Zeitraum von 5 min bis 6 Stunden bei einer Temperatur im Bereich von 4 °C bis 45 °C gemischt wird und/oder wobei Mischen der Präkomplexlösung mit der zweiten Matrix in Schritt ii) über einen Zeitraum von 5 min bis 6 Stunden bei einer Temperatur im Bereich von 4 °C bis 45 °C durchgeführt wird.

13. Verfahren nach einem der Ansprüche 11 oder 12, wobei der pH-Wert in Schritt ii) im Bereich zwischen 6 und 8, vorzugsweise zwischen 6,25 und 7,5, liegt.

14. Mikroverkapselte mikrobielle Kultur oder Zusammensetzung, die nach einem Verfahren nach einem der Ansprüche 11 bis 13 erhältlich ist.

15. Verwendung einer mikroverkapselten mikrobiellen Kultur oder einer Zusammensetzung nach einem der Ansprüche 1 bis 9 oder 14 in einem Produkt, ausgewählt aus der Gruppe, bestehend aus einem Futtermittel, einem Pflanzengesundheitsprodukt, einem Lebensmittel, einem Getränk und einem pharmazeutischen Produkt.

## Revendications

1. Culture microbienne microencapsulée, ladite culture microbienne microencapsulée comprenant :
i) une culture microbienne,
ii) une première matrice comprenant un premier composant de coacervat, et
iii) une seconde matrice comprenant un second composant de coacervat,
dans laquelle lesdits premier et second composants de coacervat sont de l'amidon d'anhydride octénylsuccinique (OSA) ou du chitosane et dans laquelle lesdits premier et second composants de coacervat ne sont pas identiques.

2. Culture microbienne microencapsulée selon la revendication 1, dans laquelle la culture microbienne est une bactérie ou une levure.

3. Culture microbienne microencapsulée selon l'une quelconque des revendications 1 ou 2, dans laquelle la culture microbienne est ou comprend des bactéries lactiques (LAB) d'un genre choisi dans le groupe constitué de *Lactobacillus, Holzapfelia, Amylolactobacillus, Bifidobacterium, Bombilactobacillus, Companilactobacillus, Lapidilactobacillus, Agrilactobacillus, Schleiferilactobacillus, Loigolactobacillus, Lacticaseibacillus, Latilactobacillus, Dellaglioa, Liquorilactobacillus, Ligilactobacillus, Lactiplantibacillus, Furfurilactobacillus, Paucilactobacillus, Limosilactobacillus, Fructilactobacillus, Acetilactobacillus, Apilactobacillus, Levilactobacillus, Secundilactobacillus, Lentilactobacillus, Leuconostoc, Pediococcus, Lactococcus, Streptococcus, Enterococcus, Bifidobacterium, Brevibacterium* et *Staphylococcus.*

4. Culture microbienne microencapsulée selon l'une quelconque des revendications précédentes, dans laquelle la culture microbienne est une culture probiotique.

5. Culture microbienne microencapsulée selon l'une quelconque des revendications précédentes, dans laquelle le rapport (% en poids/% en poids) de l'amidon d'OSA au chitosane est compris entre 75:25 et 98:2, de préférence entre 85:15 et 95:5.

6. Culture microbienne microencapsulée selon l'une quelconque des revendications précédentes, dans laquelle le rapport (% en poids/% en poids) des première et seconde matrices combinées à la culture microbienne est compris entre 0,5 et 10.

7. Culture microbienne microencapsulée selon l'une quelconque des revendications précédentes, dans laquelle la première ou la seconde matrice comprend en outre un antioxydant.

8. Culture microbienne microencapsulée selon la revendication 7, dans laquelle l'antioxydant est le citrate trisodique, la vitamine C, la vitamine E, le glutathion ou des dérivés de ceux-ci.

9. Composition comprenant la culture microbienne microencapsulée selon l'une quelconque des revendications précédentes, dans laquelle la composition comprend en outre un ou plusieurs additifs choisis dans le groupe constitué d'ingrédients de qualité alimentaire, d'ingrédients pharmaceutiques et d'excipients.

10. Produit comprenant la culture microbienne microencapsulée selon l'une quelconque des revendications 1 à 8 ou la composition selon la revendication 9, dans lequel le produit est choisi dans le groupe constitué d'un aliment pour animaux, d'un produit phytosanitaire, d'un aliment, d'une boisson et d'un produit pharmaceutique.

11. Procédé de préparation d'une culture microbienne microencapsulée selon l'une quelconque des revendications 1 à 8 ou d'une composition selon la revendication 9, ledit procédé comprenant les étapes de :
i) mélange d'une culture microbienne avec une première matrice comprenant un premier composant de coacervat, et éventuellement un ou plusieurs antioxydants, pour former une solution de pré-complexe, et
ii) mélange de la solution de pré-complexe avec une seconde matrice comprenant un second composant de coacervat, et éventuellement un ou plusieurs antioxydants, pour former une culture microbienne microencapsulée,
dans lequel lesdits premier et second composants de coacervat sont de l'amidon d'anhydride octénylsuccinique (OSA) ou du chitosane et dans lequel lesdits premier et second composants de coacervat ne sont pas identiques.

12. Procédé selon la revendication 11, dans lequel la culture microbienne de l'étape i) est mélangée avec la première matrice pendant une période comprise dans la plage de 5 min à 6 heures à une température comprise dans la plage de 4 °C à 45 °C, et/ou dans lequel le mélange de la solution de pré-complexe avec la seconde matrice à l'étape ii) est effectué pendant une période comprise dans la plage de 5 min à 6 heures à une température comprise dans la plage de 4 °C à 45 °C.

13. Procédé selon l'une quelconque des revendications 11 ou 12, dans lequel le pH à l'étape ii) est compris dans la plage comprise entre 6 et 8, de préférence entre 6,25 et 7,5.

14. Culture microbienne microencapsulée ou composition pouvant être obtenue par un procédé selon l'une quelconque des revendications 11 à 13.

15. Utilisation d'une culture microbienne microencapsulée ou d'une composition selon l'une quelconque des revendications 1 à 9 ou 14 dans un produit choisi dans le groupe constitué d'un aliment pour animaux, d'un produit phytosanitaire, d'un aliment, d'une boisson et d'un produit pharmaceutique.
